# EUROPEAN PATENT APPLICATION

(11) **EP 1 739 176 A1**
(43) Date of publication of application: **03.01.2007**
(21) Application number: 05727504.2
(22) Date of filing: 28.03.2005
(51) Int. Cl.: C12N 15/12, A61K 31/7088, A61K 48/00, A61P 21/00, A61P 25/00, A61P 25/16, A61P 25/28, A61P 29/00, A61P 35/00

(54) **DECOY NUCLEIC ACID TO SYNOVIOLIN GENE PROMOTER**

(30) Priority: 26.03.2004 JP 2004092570
(71) Applicant: Locomogene, Inc., Kanagawa 230-0046 (JP)
(72) Inventor: NAKAJIMA, Toshihiro, Kohoku Garden Hills -503, Yokohama-shi, Kanagawa 2240001 (JP); TSUTIMOCHI, Kaneyuki, Kawasaki-shi, Kanagawa 2140035 (JP); YAGISHITA, Naoko, Yokohama-shi, Kanagawa 234-0053 (JP); YAMASAKI, Satoshi, Ishikawazaka Manshon 305, Yokohama-shi, Kanagawa 2250003 (JP); KATO, Yukihiro, Yamato-shi, Kanagawa 2420028 (JP); AMANO, Tetsuya, Kawasaki-shi, Kanagawa 2140005 (JP); TAMITSU, Kaori, Koganei-shi, Tokyo 1840012 (JP)
(74) Representative: Woods, Geoffrey Corlett
(86) International application number: PCT/JP2005/006527
(87) International publication number: WO 2005/093067

(57) **Abstract**

The present invention provides a decoy nucleic acid for the Synoviolin gene promoter. Also provided is the decoy nucleic acid can inhibit the promoter activity by binding to the transcription factor of the Synoviolin gene promoter. It also provides a decoy nucleic acid as expressed in (a) a decoy nucleic acid consisting of a nucleotide sequence as shown in SEQ ID NO: 11 or 12; and it also provides (b) a decoy nucleic acid consisting of a nucleotide sequence as shown in SEQ ID NO: 11 or 12 having deletion, substitution or addition of one or several nucleotides and has the function of inhibiting Synoviolin gene promoter activity.

## Description

### TECHNICAL FIELD

The present invention relates to a decoy nucleic acid to Synoviolin gene promoter.

### BACKGROUND ART

Rheumatoid arthritis (hereinafter referred to as RA) is a systemic chronic inflammatory disease wherein hyperplasia is seen in the synovial tissue of joints. The inventors identified the Synoviolin gene as an essential gene to hyperplasia of the synovial tissue (WO 02/052007).

The Synoviolin gene encodes Synoviolin and is a membrane protein cloned by immunoscreening, using anti-synovial cell antibodies, from the synovial cell library of the RA patients. When a Synoviolin molecule is overexpressed in the mouse, hyperplasia of synovial membranes, bone and cartilage disruption were found in joints, thus exhibiting symptoms resembling those of rheumatoid arthritis. This suggested the contribution of Synoviolin to the generation, differentiation, regeneration and metabolism of cartilage and bone tissue. In addition, Synoviolin was recently discovered to be involved in the onset of fibrosis, cancers and cerebral neural diseases (Genes Dev. 2003 Vol. 17: p. 2436-49).

The level of Synoviolin expression was thought to be important in the aforementioned diseases. In order to search for the transcriptional regions of Synoviolin promoters, the promoter activity was investigated with respect to the fragments in different lengths that were obtained by cutting the upstream promoter regions of the mouse Synoviolin gene. As a result, a core region was specified and it was confirmed to have interactions with the transcription factor (Oncogene, 2000 Vol. 19: p. 6533-48).

### DISCLOSURE OF THE INVENTION

The purpose of the present invention is to provide a decoy nucleic acid for the site that encodes the transcriptional regulatory region of Synoviolin gene promoter.

The inventors earnestly investigated the aforementioned problems and succeeded in preparing a decoy nucleic acid for the aforementioned site of the Synoviolin gene. This finding led us to achieve the present invention.

That is, the present invention is as follows:
(1) A decoy nucleic acid, which can inhibit promoter activity by binding to the transcription factor of the Synoviolin gene promoter.
   The decoy nucleic acids of the present invention, for example, include the following (a) or (b):
   (a) A decoy nucleic acid consisting of a nucleic acid sequence as shown in SEQ ID NO: 11 or 12
   (b) A decoy nucleic acid consisting of a nucleic acid sequence as shown in SEQ ID NO: 11 or 12 having deletion, substitution or addition of one or several nucleic acids, and has a function of inhibiting Synoviolin gene promoter activity.

   The decoy nucleic acid of the present invention may be the following (a) or (b):
   (a) A decoy nucleic acid consisting of a nucleic acid sequence as shown in SEQ ID NO: 11 and 12
   (b) A decoy nucleic acid consisting of a nucleic acid sequence as shown in SEQ ID NO: 11 and 12 having deletion, substitution or addition of one or several nucleic acids, and has a function of inhibiting Synoviolin gene promoter activity.

   As a function of inhibiting the Synoviolin gene promoter activity, for example, a function of binding with a transcription factor of the Synoviolin gene promoter is given.
   The decoy nucleic acid of the present invention can be designed based on a nucleotide sequence at the transcription factor binding site selected from a group consisting of EBS (Ets binding site), SBS (Sp1 binding site) and ABS (AML binding site).
   The decoy nucleic acids of the present invention induce apoptosis in a synovial cell or a cancer cell.
(2) Pharmaceutical composition containing the aforementioned decoy nucleic acid for treating and preventing the diseases attributed to the expression of the Synoviolin gene.
   The pharmaceutical composition of the present invention further contains pharmaceutically acceptable carrier. At least one indication selected from the group consisting of rheumatoid arthritis, fibrosis, cancers and cerebral neural diseases is the target indication for the pharmaceutical composition of the present invention.
(3) A method of inhibiting the transcription activity of Synoviolin transcription factor using said decoy nucleic acid.
(4) A method of inhibiting the Synoviolin promoter activity using said decoy nucleic acid.
(5) A method of suppressing the expression of Synoviolin by inhibiting the Synoviolin promoter activity using said decoy nucleic acid.
(6) A method of inducing apoptosis in a synovial cell or a cancer cell using said decoy nucleic acid.

### BRIEF DESCRIPTION OF THE DRAWINGS

Fig. 1A is a diagram showing the activity of the truncated type Synoviolin promoter.
Fig. 1B is a diagram showing the activity of the truncated type Synoviolin promoter.
Fig. 1C is a diagram indicating the Synoviolin promoter Ets binding site and its core region.
Fig. 1D is a diagram showing the transcriptional activity when introducing mutations into the promoter.
Fig. 2A is a photograph showing the results when performing a gel shift assay.
Fig. 2B is a photograph showing the results when performing a gel shift assay.
Fig. 2C is a photograph showing the results when performing a gel shift assay.
Fig. 2D is a photograph showing the results when performing a gel shift assay.
Fig. 3A is a diagram showing the transcription activity of the Synoviolin promoter.
Fig. 3B is a diagram showing the transcription activity of the Synoviolin promoter.
Fig. 3C is a diagram showing the transcription activity of the Synoviolin promoter in the NIH3T3 cell knocked out using RNAi.
Fig. 4A is a plasmid construction diagram when LacZ binds to 2.2 k and 1 k Synoviolin promoters.
Fig. 4B is a photograph showing the expression of the Synoviolin promoter in a mouse embryo.
Fig. 4C is a photograph showing the expression of the Synoviolin promoter in a mouse embryo.
Fig. 5 is a diagram showing the sequence of a decoy nucleic acid.
Fig. 6 is a diagram showing the time schedule for the confirmation tests on inhibition of expression of Synoviolin in the synovial cells.
Fig. 7A is a photograph showing the expression of the Synoviolin promoter in the RA synovial cells.
Fig. 7B is the results of WST-8 assay showing the inhibition of the Synoviolin expression in the synovial cell.
Fig. 7C is a photograph showing the results of the expression of Synoviolin in the synovial cells confirmed by the Western Blotting.
Fig. 8A is a diagram showing the results of luciferase assay.
Fig. 8B is a photograph showing apoptosis in the NIH3T3 cells wherein Synoviolin has been knocked down using a decoy ODN and the results of the Western Blotting.
Fig. 8C is a photograph showing apoptosis in the NIH3T3 cells wherein Synoviolin has been knocked down using RNAi and the results of the Western Blotting.
Fig. 9A is a photograph showing the inhibition of the expression of Synoviolin by EBS-1 decoy ODN.
Fig. 9B shows apoptosis by the EBS-1 decoy ODN treatment in the NIH3T3 cells wherein Synoviolin has been overexpressed.
Fig. 10 is a diagram showing the inhibitory effect on Synoviolin RNA in the RA synovial cells using EBS-1 decoy.

### BEST MODE FOR CARRYING OUT THE INVENTION

The present invention is explained in detail below.

The present invention is characterized by providing a decoy nucleic acid for the site of encoding the transcriptional regulatory region of the Synoviolin gene promoter.

### 1. Overview

The inventors have been analyzing RA primarily with respect to Synoviolin. Recently, it was discovered in the analysis using Synoviolin-knockout mouse that the level of Synoviolin having ERAD functions is related to the threshold value of the apoptosis induction by ER stress.

In order to elucidate the transcription mechanism regulating the amount of Synoviolin that determines sensitivity of apoptosis induction, we performed a promoter analysis and identified the Ets binding site (EBS: Ets binding site) responsible for the structural expression of Synoviolin using mouse cell lines and mouse embryos. It was proved that this element is essential for the in vivo expression of Synoviolin. Ets is a transcription factor that has been conserved from yeast to human. The Ets domain forms more than 30 kinds of families and is mostly responsible for the transcription activity of molecules carrying out differentiation, proliferation and apoptosis (D.K. Watoson and A. Seth: Oncogene, Review Issue. Dec. 18; 19 (1955); 2000). Also in the NIH3T3 cells, it was demonstrated that a GABPα/β complex, member of the Ets family, is involved in transcriptional regulation via EBS.

The expression of Synoviolin is ubiquitous according to an analysis of the expression of hetero knockout mouse (LacZ knock in mouse), Northern and Western, suggesting that the expression of Synoviolin is required in many cells. This was hypothesized from the fact that the hyperfunction of systemic apoptosis was fatal to embryos in Synoviolin knockout mouse. However, its expression fluctuated, and in particular, its expression was strong in certain cells (pancreas, testis and neural cells) showing a high secreting ability. This suggested that the expression of Synoviolin is very highly required by a part of the cells.

The transcriptional regulation mechanism consists of (i) a constitutive gene expression containing complexes of general transcription factor and (ii) an inducible gene expression to certain stimulants. There is no TATA box or initiator sequence in the Synoviolin promoter. In the case of such a promoter construction, transcription factors such as SP1, Ets families, and the like, are important in transcriptional induction (Rudge, Exp Cell Res. Mar 10:274 (1): 45-55, 2002).

Actual examples of transcription factors include GABPα, GABPβ, a complex of GABPα and GABPβ, Ets1, Tel, Fli-1, etc.

GABPα is one of the Ets families having an Ets domain and is a protein having 454 amino acids. GABPα is expressed ubiquitously in cells and forms a hetero dimer with GABPβ via the Ets domain. Moreover, it is known that GABPα uses two Ets binding sites to form a hetero tetramer. Also, there are some of these characteristics in the transcriptional regulation of target genes. First of all, GABPα has a DNA binding ability by the Ets domain, but it does not have transcription activation ability. In contrast, GABPβ does not have a DNA binding ability, but it induces a transcription activity by forming a dimer with GABPα and exhibits a further higher transcription activation ability by forming a hetero tetramer (Yu M. J Biol Chem Nov 14; 272 (46): 29060-7, 1997). Also, GABPα can act as an initiator in the expression of genes having no TATA box and genes having multiple transcription initiation points (Yu M. J Biol Chem Nov 14: 272 (46): 29060-7, 1997; Kamura T. J Biol Chem Apr 25; 272 (17); 11361-8, 1997). Moreover, despite the fact that the expression of GABPα is ubiquitous, it acts synergistically with the transcription factor of the partner binding at other sites to carry the expression of genes concerning cell-specific differentiation and proliferation (Schaeffer L, EMBO J. Jun 1; 17 (11): 3078-90, 1998).

GABPβ is not in the Ets families, but it is a cofactor of GABPα having 382 amino acids. It forms a hetero dimer with GABPα via an Ankyrin repeated sequence and has a transcription activity region.

Ets1 is a human homolog of v-Ets. It was discovered in 1983 as a carcinogenic gene of the retrovirus E26, causing erythroblastosis in chickens, and is a protein containing 441 amino acids.

Tel is a protein containing 452 amino acids and is reported to act for transcriptional inhibition among the Ets families. Clinically, it forms a fusion gene with AML 1 by t (12; 21) chromosomal translocation and it is known to cause leukemia.

Fli-1 consists of 452 amino acids and forms a fusion gene with EWS by t (11; 22) chromosomal translocation and it is known to cause Ewing's sarcoma.

The Ets family is a transcription factor having a domain called Ets preserved from the yeast to human. This domain formed more than 30 kinds of families and the majority carries the molecular transcription activity associated with differentiation, proliferation and apoptosis (D.K. Watoson and A. Seth; Oncogene. review issue. Dec. 18; 19 (55); 2000).

In order to obtain a promoter region, there is a method of cutting using a restriction enzyme from the Synoviolin gene, or a mouse or a human genome sequence or the like. However, since a convenient restriction enzyme recognition sites is not always generally present at an appropriate location, a desirable promoter region can be obtained by amplifying by PCR by using a primer wherein a restriction enzyme recognition site has been incorporated in advance. Also, based on the known nucleic acid sequence information in the promoter region, it is possible to chemically synthesize a desirable promoter region. In this case, the presence of ABS and SBS is suggested as transcription factor binding sites besides EBS. In fact, whether the transcription factor binds at such a position is determined by mutation at each site to reduce its transcription activity or by confirming by gel shift assay (electrophoretic mobility shift assay: EMSA) that the transcription factor binds with the original probe, but it does not bind after the introduction of one base mutation.

Here, SEQ ID NO: 1 and SEQ ID NO: 2 represents the full length promoter sequence of the mouse Synoviolin gene and the full length promoter sequence of the human Synoviolin gene, respectively.

Also, SEQ ID NO: 8 represent a nucleic acid sequence encoding EBS-1 that is a promoter core region, SEQ ID NO: 9 and SEQ ID NO. 10 represent a nucleic acid sequence encoding ABS-1 and SBS-1, respectively.

### 2. Decoy nucleic acid

The present invention relates to a decoy nucleic acid (decoy oligonucleotide) that binds to the aforementioned transcription factor to be able to inhibit the promoter activity. A decoy nucleic acid in the present invention implies a short decoy nucleic acid including the binding site for a transcription factor. If this nucleic acid is transfected into the cell, transcription factor binds to this nucleic acid competitively to inhibit binding to the original binding site on the genome the transcription factor. As a result, expression of the transcription factor is inhibited. Typically, a decoy nucleic acid is a nucleic acid and its analogs, which contains at least one nucleic acid sequence that can bind to the target binding sequence.

Desirable examples of decoy nucleic acid are as follows. For example, a nucleic acid capable of binding to a transcription factor that binds to the promoter's EBS-1; a nucleic acid capable of binding to a transcription factor that binds to the promoter's ABS; a nucleic acid capable of binding to a transcription factor that binds to the promoter's SBS-1; oligonucleotides containing their complementary base pairs, their mutants or nucleic acids containing them in the molecule. Decoy nucleic acids can be designed based on the above-mentioned sequences, EBS-1, ABS or SBS-1, by forming a single strand or double strands comprising of its complementary strand. The length is not particularly limited, but a desirable length ranges from 15 to 60 bases and preferably from 20 to 30 bases.

In the present invention, a nucleic acid capable of binding to a transcription factor binding to EBS-1 (SEQ ID NO: 11) and/or its complementary strand (SEQ ID NO: 12) can be used preferably as a decoy nucleic acid.

Nucleic acids can be DNA or RNA, or the nucleic acid can contain modified nucleic acids and/or a pseudo nucleic acid. The nucleic acids containing nucleic acid, mutant thereof, or nucleic acid containing these within the molecule, can be made of a single strand or double strands. In addition, it can be cyclic or linear. A mutant is made of a base sequence from which one to several bases in the aforementioned decoy nucleic acid (For example, 1 to 10, 1 to 5, or 1 to 2, etc.) are deleted, substituted, or added, and it has a function of inhibiting the promoter activity of the synoviolin gene, namely it is called a nucleic acid having a function of binding with a transcription factor. It can be a nucleic acid containing one or more of the aforementioned base sequences.

The decoy nucleic acid used in the present invention can be produced by a chemical synthesis or a biochemical synthesis known in the art. For example, a nucleic acid synthesis method using a common DNA synthesis device can be employed as a gene recombinant technology. In addition, after separating or synthesizing a base sequence to be a template, a PCR method or a gene amplification method can be used. Moreover, the nucleic acid obtained according to these methods can be cleaved with a restriction enzyme, and a desired nucleic acid can be manufactured by linking the nucleic acid using a DNA ligase. Moreover, in order to obtain a more stable decoy nucleic acid in the cell, chemical modifications such as alkylation and acylation can be added to the base sequence. A method of preparing a mutant from the decoy nucleic acid, a method that is known in the art, can be employed. For example, it can be synthesized by a site-directed mutagenesis method. A site-directed mutagenesis method is known in the art and a commercial kit can be purchased. For example, the following commercial kits can be used: GeneTailor™ Site-Directed Mutagenesis System (Invitrogen Corp.), TaKaRa Site-Directed Mutagenesis System (Mutan-K, Mutan-Super Express Km, etc. (Takara Bio Corp)).

For an analysis of the transcription activity for promoters when using a decoy nucleic acid, the following generally known method can be employed: e.g., Luciferase assay, gel shift assay, CAT assay, a staining method using Annexin V which is a specific marker to apoptosis, Western blotting, FACS analysis, RT-PCR, etc. A kit is also commercially available to carry out these assays (e.g., Promega Dual Luciferase Assay Kit.).

For example, in the case of the Luciferase assay, a firefly luciferase gene is ligated as a reporter upstream to the transcription initiation point of the target gene. In order to correct intercellular transfection efficacy for the assay, a cytomegalovirus (CMV) β-galactosidase (β-gal) gene acting as a reporter can be introduced to the cell along with the vector ligated downstream to the promoter at the same time. For the transfection into the cell, for example, a calcium phosphate method can be employed. If a vector is transfected into the cells, the cells can be cultured for a specified time and then recovered. Subsequently, the cells are disrupted by freezing and fusing, luciferase and β-galactosidase activity was measured using a fixed amount of cell extract.

According to these analyses, if decoy nucleic acids are used, the transcription activity of Synoviolin is inhibited and apoptosis inducing in synovial cells and cancer cells are shown.

### 3. Pharmaceutical composition containing decoy nucleic acid

The present invention relates to a pharmaceutical composition containing one or more said decoy nucleic acids for treating or preventing the diseases attributed to the expression of the synoviolin gene. The decoy nucleic acids of the present invention can be used as a pharmaceutical composition of the present invention as long as they have a binding activity to the target binding sequences.

Applicable diseases of the pharmaceutical composition of the present invention include diseases attributed to cellular hyperplasia such as RA, fibrosis, cancers, and cerebral neural diseases. When pharmaceutical composition of the present invention is applied to these diseases, said diseases can be present singly, or multiple diseases can be associated.

When the pharmaceutical composition of the present invention is used as a cancer treatment drug, types of cancers are not limited, for example, target cancers include brain tumors, tongue cancer, pharyngeal cancer, lung cancer, breast cancer, esophageal cancer, gastric cancer, pancreatic cancer, gall bladder cancer, biliary tract carcinoma, duodenum cancer, colon cancer, liver cancer, uterine cancer, ovarian cancer, prostate cancer, renal cancer, bladder cancer, rhabdomyosarcoma, fibrosarcoma, osteosarcoma, chondrosarcoma, skin cancer, various kinds of leukemias (e.g., acute myelogenous leukemia, acute lymphatic leukemia, chronic myelogenous leukemia, chronic lymphatic leukemia, adult T-cell leukemia, malignant lymphoma), etc.

The aforementioned cancers include the primary lesion or metastasis. Other diseases can be associated.

Cerebral neural diseases include Alzheimer's disease, Parkinson's disease, and polyglutamine disease.

The pharmaceutical composition of the present invention can be used in such a form that decoys can be incorporated into the cellular lesions or into the tissue cells.

The mode of administration of the pharmaceutical composition of the present invention containing decoy nucleic acids can be either an oral or a parenteral route. In the case of oral administration, an appropriate drug form can be selected from tablets, pearls, sugarcoated tablets, capsules, liquid agents, gels, syrups, slurries and suspensions. In the case of parenteral administration, via pulmonary administration types (e.g., using a nebulizer, etc.), via nasal administration types, subcutaneous injection types (e.g., ointments, cream agents), and injection types are available. In the case of injection types, agents can be administered systemically or locally, directly or indirectly to the diseased areas via various drip fusions such as intravenous injection, intramuscular injection, intraperitoneal injection and subcutaneous injection.

When the pharmaceutical composition of the present invention is used as a gene therapy, in addition to direct. administration by injection of the composition, a method of administering a vector incorporating a nucleic acid is available. As the aforementioned vectors, adenovirus vector, adeno-associated virus vector, herpes virus vector, vaccinia virus vector, retrovirus vector, lentivirus vector, and the like are available. Use of these viral vectors makes administration more efficient.

A pharmaceutical composition of the present invention can be introduced into a phospholipid vesicle, such as a liposome, and the vesicle can be administered. A vesicle retaining a pharmaceutical composition of the present invention is introduced into a specific cell by the lipofection method. The cells obtained are then administered systemically intravenously or intra-arterially. They can be administered locally, for example, to the brain. In order to introduce the pharmaceutical composition of the present invention into the target tissues or organs, commercial gene transfection kits (e.g., Adeno Express: Clontech Corp.) can be used. As lipids to form a liposome structure, phospholipids, cholesterols and nitrolipids can be used. In general, phospholipids are suitable. Natural phospholipids such as phosphatidylcholine, phosphatidylserine, phosphatidylglycerol, phosphatidylinositol, phosphatidylethanolamine, phosphatidic acid, cardiolipin, sphingomyeline, egg yolk lecithin, soybean lecithin, lysolecithin; or their hydrogenated products prepared by conventional method, are available. In addition, following synthetic phospholipids can be used: dicetyl phosphate, distearoylphosphatidylcholine, dipalmitoylphosphatidylcholine, dipalmitoylphosphatidylethanolamine, dipalmitoylphosphatidylserine, eleostearoylphosphatidylcholine, and eleostearoylphosphatidylethanolamine, etc.

A method of producing liposome is not particularly limited, as long as a decoy can be retained. The liposome can be prepared by conventional methods including a reverse phase vaporization method (Szoka, F. et al., Biochim. Biophys. Acta, Vol. 601 559 (1980)), an ether injection method (Deamer, D.W.: Ann. N.Y. Acad. Sci., Vol. 308 250 (1978)), and a surfactant method (Brunner, J. et al.: Biochim. Biophys. Acta, Vol. 455 322 (1976)).

Lipids containing these phospholipids can be used singly, or two or more kinds can be combined. In this case, if those containing an atomic group having cationic groups, such as ethanolamine and choline, in the molecule are used, the degree of association of decoy nucleic acid that is electrically negative can be increased. Besides major phospholipids used when forming these liposomes, additives that are generally known as additives for liposome formation, such as cholesterol, stearylamine, and α-tocopherol, etc., can be used. To the liposomes obtained above, membrane fusion-promoting substances such as the Sendai virus, inactivated Sendai virus, membrane fusion-promoting proteins purified from Sendai virus, polyethylene glycol, etc., can be used in order to promote intake into the cells in a diseased region or the cells in a target tissue.

The pharmaceutical composition of the present invention can be formulated by a conventional method and can contain pharmaceutically acceptable carriers. Such carriers can be additives or the following additives are available: water, pharmaceutically acceptable organic solvents, collagen, polyvinyl alcohol, polyvinylpyrrolidone, carboxyvinyl polymer, sodium carboxymethylcellulose, sodium polyacrylate, sodium alginate, water-soluble dextran, sodium carboxymethyl starch, pectin, methylcellulose, ethyl cellulose, xanthan gum, gum arabic, casein, agar, polyethylene glycol, diglycerin, glycerin, propylene glycol, vaseline, paraffin, stearyl alcohol, stearic acid, human serum albumin, mannitol, sorbitol, lactose, and surfactants that are acceptable as pharmaceutical additives.

The aforementioned additives can be selected singly or in combination according to the types of formulas as therapeutic agent of the present invention. For example, when used as an injection formula, a purified decoy nucleic acid is dissolved in a solvent (e.g., saline, buffer solution, glucose solution, etc.) and then mixed with Tween 80, Tween 20, gelatin, and human serum albumin, etc. Alternatively, it can be freeze-dried form to be dissolved before use. As an excipient for freeze dry, the following materials are available: sugars such as mannitol, glucose, lactose, sucrose, mannitol and sorbitol etc.; starch such as corn, wheat, rice, potato and other vegetable starch; celluloses such as methyl cellulose, hydroxypropylmethyl cellulose, or sodium carboxymethylcellulose; rubbers such as gum Arabic, traganto rubber; gelatin and collagen, etc. If desirable, disintegrants or solubilizers, such as cross-linked polyvinyl pyrrolidone, agar, alginic acid or its salts (e.g., sodium alginate) are available.

Doses of the pharmaceutical composition of the present invention vary with age, sex, symptoms, administration routes, frequency of administration, and types of formulas. A method of administration is appropriately selected based on patient's age and symptom. An effective dose is the amount of a decoy nucleic acid that is required for reducing symptoms of the diseases and conditions. For example, the therapeutic effect and toxicity of the nucleic acid may be determined through standard pharmacological procedures in cell cultures or experimental animals, such as ED₅₀ (therapeutically effective dose at 50% of the population) or LD₅₀ (lethal dose at 50% of the population).

A dose ratio between the therapeutic effect and the toxicity effect is therapeutically efficient and is expressed as ED₅₀/LD₅₀. A single dosage of the pharmaceutical composition of the present invention ranges from 0.1µg to 100 mg per kg bodyweight and preferably from 1 to 10 µg. However, the aforementioned treatment agent is not limited by these dosages. For example, if an adeno virus is administered, a single daily dose is approximately 10⁶ to 10¹³ pfu and is administered with an interval of 1 week to 8 weeks. However, the pharmaceutical composition of the present invention is not limited by these dosages.

This invention will be described in detail with reference to the examples. However, this invention will not be limited by these examples.

### [Example 1]

This example relates to plasmid construction for the preparation of the synoviolin promoters.

Using a mouse (C57B1/6) genome, approximately 7.5 k of genome from 5' to 3' containing synoviolin gene was subcloned into SyB/pBluescript. Subsequently, the promoter region of the synoviolin gene was treated with XhoI and NcoI to extract an approximately 2.2 k fragment, which was inserted into PGV-B2 (TOYO INK GROUP Corp.) (SyG-2.2 k). This approximately 2.2 k fragment was considered to be a full-length promoter (SEQ ID NO: 1). A partial region of the full-length promoter was deleted from the 5'-side to prepare a construct with a shortened promoter region. The promoters prepared are listed below in Table 1.

**Table 1. List of the promoters prepared (Mouse)**

| Title | Regions* | Regions of the nucleotide sequence shown in SEQ ID NO: 1 |
|---|---|---|
| -2201/+843 (Full-length) | Using the TS (Transcription initiation site) as a starting point (+1), a region consisting of upstream (5'-end) 2201 nucleotides and downstream (3'-end) 843 nucleotides | 1~3043 |
| -1233/+843 | Using the TS as a starting point, a region consisting of upstream 1233 nucleotides and downstream 843 nucleotides | 969~3043 |
| -1060/+843 | Using the TS as a starting point, a region consisting of upstream 1060 nucleotides and downstream 843 nucleotides | 1142~3043 |
| -503/+843 | Using the TS as a starting point, a region consisting of upstream 503 nucleotides and downstream 843 nucleotides | 1699~3043 |
| -322/+843 | Using the TS as a starting point, a region consisting of upstream 322 nucleotides and downstream 843 nucleotides | 1880~3043 |
| -200/+843 | Using the TS as a starting point, a region consisting of upstream 200 nucleotides and downstream 843 nucleotides | 2002~3043 |
| -108/+843 | Using the TS as a starting point, a region consisting of upstream 108 nucleotides and downstream 843 nucleotides | 2094~3043 |
| -84/+843 | Using the TS as a starting point, a region consisting of upstream 84 nucleotides and downstream 843 nucleotides | 2118~3043 |
| -73/+843 | Using the TS as a starting point, a region consisting of upstream 73 nucleotides and downstream 843 nucleotides | 2129~3043 |
| -65/+843 | Using the TS as a starting point, a region consisting of upstream 65 nucleotides and downstream 843 nucleotides | 2137~3043 |
| -39/+843 | Using the TS as a starting point, a region consisting of upstream 39 nucleotides and downstream 843 nucleotides | 2163~3043 |
| -10/+843 | Using the TS as a starting point, a region consisting of upstream 10 nucleotides and downstream 843 nucleotides | 2191~3043 |

| | | |
|---|---|---|
| In the region, one nucleotide at 5'-end of the TS when facing upstream (5'-end) (t at 2201^{st} of SEQ ID NO: 1) is counted as -1 and the TS when facing downstream (3'-end) is counted as + 1. | | |

The truncated type promoters of human synoviolin gene can be prepared similarly in the case of mouse (SEQ ID NO: 2). The positions of excisions are shown in Table 2.

**Table 2. List of the promoters prepared (Human)**

| Title | Regions* | Regions of the nucleotide sequence shown in SEQ ID NO: 2 |
|---|---|---|
| -2201/+892 (Full-length) | Using the TS (Transcription initiation site) as a starting point (+1), a region consisting of upstream (5'-end) 2201 nucleotides and downstream (3'-end) 892 nucleotides | 1~3092 |
| -1233/+892 | Using the TS as a starting point, a region consisting of upstream 1233 nucleotides and downstream 892 nucleotides | 969~3092 |
| -1060/+892 | Using the TS as a starting point, a region consisting of upstream 1060 nucleotides and downstream 892 nucleotides | 1142~3092 |
| -503/+892 | Using the TS as a starting point, a region consisting of upstream 503 nucleotides and downstream 892 nucleotides | 1699~3092 |
| -322/+892 | Using the TS as a starting point, a region consisting of upstream 322 nucleotides and downstream 892 nucleotides | 1880~3092 |
| -200/+892 | Using the TS as a starting point, a region consisting of upstream 200 nucleotides and downstream 892 nucleotides | 2002~3092 |
| -108/+892 | Using the TS as a starting point, a region consisting of upstream 108 nucleotides and downstream 892 nucleotides | 2094~3092 |
| -84/+892 | Using the TS as a starting point, a region consisting of upstream 84 nucleotides and downstream 892 nucleotides | 2118~3092 |
| -73/+892 | Using the TS as a starting point, a region consisting of upstream 73 nucleotides and downstream 892 nucleotides | 2129~3092 |
| -65/+892 | Using the TS as a starting point, a region consisting of upstream 65 nucleotides and downstream 892 nucleotides | 2137~3092 |
| -39/+892 | Using the TS as a starting point, a region consisting of upstream 39 nucleotides and downstream 892 nucleotides | 2163~3092 |
| -10/+892 | Using the TS as a starting point, a region consisting of upstream 10 nucleotides and downstream 892 nucleotides | 2191~3092 |

| | | |
|---|---|---|
| * In the region, one nucleotide at 5'-end of the TS when facing upstream (5'-end) (t at 2201^{st} of SEQ ID NO: 1) is counted as -1 and the TS when facing downstream (3'-end) is counted as +1. | | |

In order to prepare mutants of the aforementioned promoters derived from a mouse, SyG-2.2 kG-76TBV2 was prepared using a site-directed mutagenesis method by overlap elongation (Molecular cloning, CSHL Press, 3rd edition, 2001, Chapter 13). The primers used are shown below:
1. EBS-1m (G-76T): GCGCCGCCGTAAGTGAGGT (SEQ ID NO: 3)
2. ABSm (G-68T): AAGTGAGTTGTCTTACCCCC (SEQ ID NO: 4)
3. SBS-1m (G-92A, C-91A): ACTCCGCCAAGCCCCGCGCC (SEQ ID NO: 5)
   PCR was performed using a reaction composition solution containing 1 pmol SyB/pBluescript, 100 pmol primer, 0.2 mM dNTPs, 5U polymerase, 10 mM Tris-HCl (pH 8.3), and 50 mM KCl in a 50 µl reaction solution under the following PCR conditions:
   PCR Conditions:
      Step 1: 94°C for 1 min. → (94°C for 30 sec. → 55°C for 30 sec. → 72°C for 1 min.) x 25 times
      Step 2: 1^{st} cycle: 94°C for 1 min. → 55°C for 30 sec. → cooled to 30°C for a period of 29 min. → 30°C for 1 min. → increased to 72°C for a period of 9 min. → 72°C for 1 min. → (94°C for 30 sec. → 55°C for 30 sec. → 72°C for 1 min.) x 25 times

EBS-1m (G-76T) is a primer to mutate G at the 2125^{th} of the nucleotide sequence shown in SEQ ID NO: 1 (76^{th} (-76) upstream from TS) to T. ABSm (G-68T) is a primer to mutate G at the 2133^{rd} of the nucleotide sequence shown in SEQ ID NO: 1 (68^{th} (-68) upstream from TS) to T. SBS-1m (G-92A, C-91A) is a primer to mutate C at the 2112^{nd} of the nucleotide sequence shown in SEQ ID NO: 1 (-91^{st} from TS) to A and to mutate G at the 2111^{th} of the nucleotide sequence shown in SEQ ID NO: 1 (-92^{nd} from TS) to A.

### [Example 2]

This is an example relating to the functional analysis of the synoviolin promoters.

In order to clarify the regulatory mechanism of the quantity of Syoviolin, a promoter analysis was performed. It has been known that Synoviolin is expressed ubiquitously based on the analysis of transgenic mouse lines (LacZ knock-in) and the results of Northern and Western blotting.

First, after cloning synoviolin promoters, the region containing 2.2 k from the translation initiation site was bound to the luciferase vector. Using various cells (as shown below), the transcription activities were investigated by constructions deleted from the upstream side. The cells were cultured using a DMEM (Dulbecco's Modified Eagle's Medium, Sigma D6046) medium supplemented with 10% inactivated bovine fetal serum (Life Technologies, Inc.).

### Cells used:

ATDC5: mouse tetracarcinoma cell AT805-derived subline, specific to cartridge and pigment cells, alkaline phosphatase positive.
HEK293: Human fetal kidney-derived cells
NIH3T3: Mouse fetus-derived fibroblasts.

Transfection and reporter assay were performed as follows.

Cells were prepared on a 24-well plate at 2 x 10⁴/well. After 24 hours, using a FUGENE6 Kit, the expression was performed according to the kit's manual (Biochem. Roche Corp.).

For correction of transfection efficiency, using each 50 ng of CMV-β-gal, only the full vector was placed to sort the total volume to 200 ng.

After transfection, proteins were recovered in 30 hours. After discarding the medium, the cells were washed with PBS and cell lysate was recovered using 100 µl of Passive Lysis Buffer ™ (Promega Corp.). Subsequently, 20 µl of the cell lysate was transferred to a 96-well plate and the Luciferase activity was measured with a luminometer. Furthermore, after measuring the β-gal activity by a plate reader, the Luc value was divided by the β-gal value for corrections. In this case, β-gal staining was performed as follows. That is, β-galactosidase was stained using X-gal (5-bromo-4-chloro-3-indolyl-β-D galactopyranoside, Sigma Corp.). Embryos were fixed in 4% paraformaldehyde for 20 minutes and dipped in an X-gal solution, then stained at 37°C for 12 to 24 hours. After staining, the specimens were washed with PBS and again fixed with 4% paraformaldehyde.

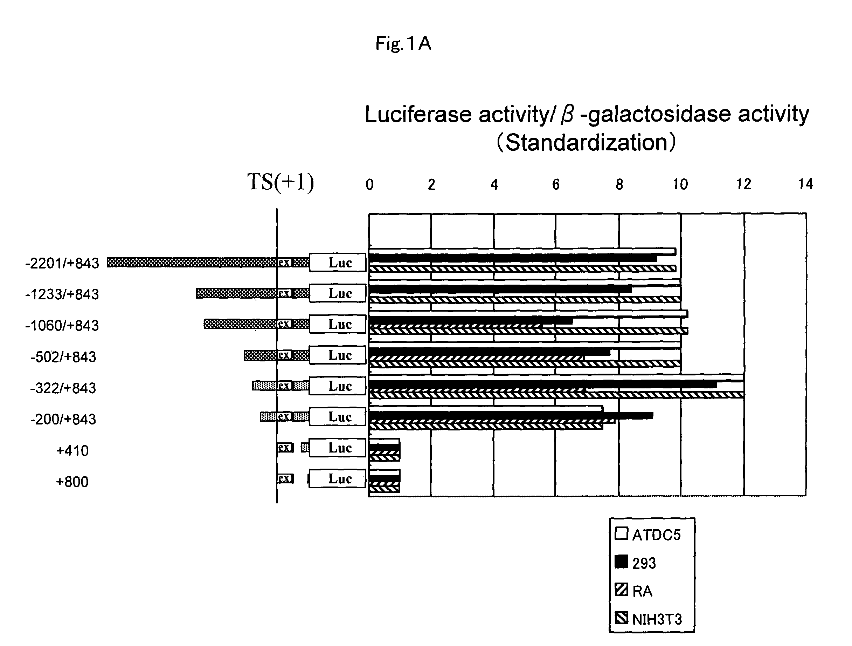

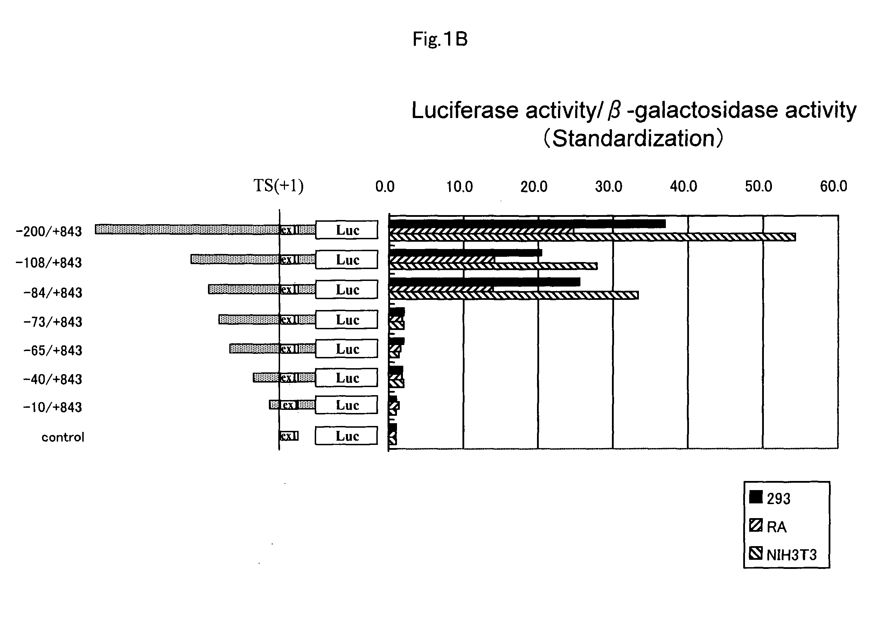

The results showed that the transcription activity declined 10% to 30% at 12 nucleotides from -84 to -73bp (Fig. 1 A and B).

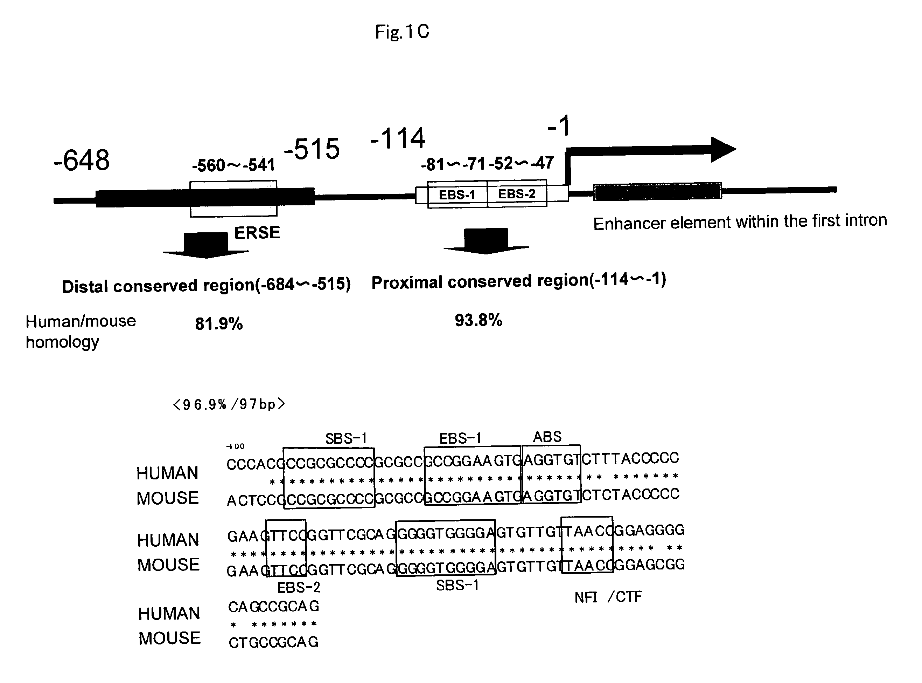

In the region from -114 to -1 containing this region, the mouse indicated 94% homology to that of a human and 100% homology at 12 nucleotides. As a result of computer-assisted bioinformatics analysis software (http://www.cbrc.jp/research/db/TFSEARCHJ.html), the EBS (Ets binding side) was found to be present at 12 nucleotides (Fig. 1C).

Subsequently, the inventors introduced mutation into the transcription factor binding sequence in the surrounding domains including this region, and the effects of the site on transcription were investigated using several cell systems.

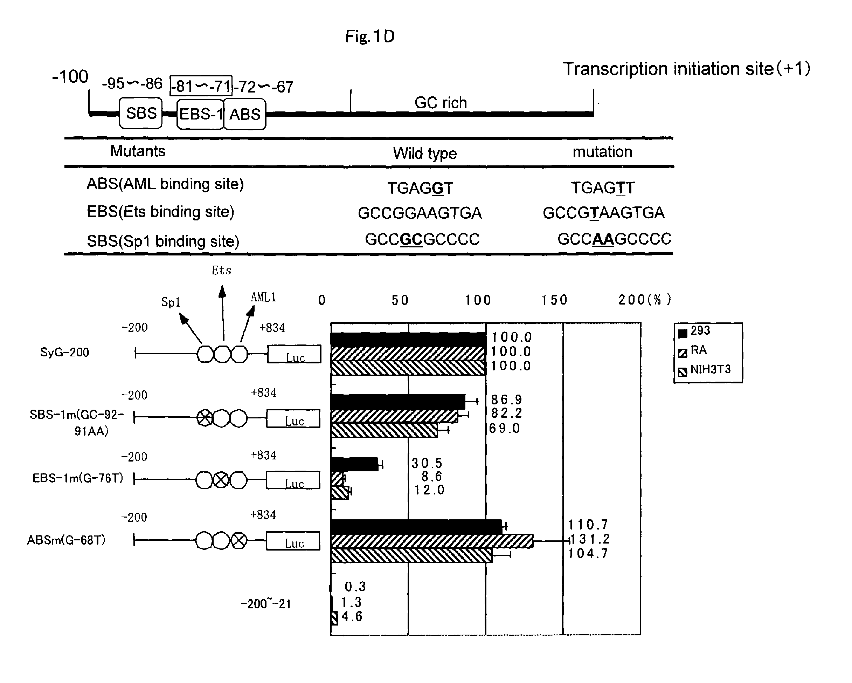

As seen in Fig. 1D, with a point mutation of EBS, the activity was found to be reduced from about 9% to 40% in almost all cell systems. As a result, the EBS was considered to be an essential element for carrying out the constitutive expression of Synoviolin (hereinafter referred as to EBS-1).

### [Example 3]

In this example, a transcription factor binding to EBS-1 (from -89 to -65) was identified.

An Ets family forms more than 30 kinds of families, and contains Ets domains that are all DNA-binding domains. The center sequence thereof is a GGAA and the subsequent sequence to this sequence was presumed to be very important for binding to this sequence.

Initially, using a probe containing 12-nucleotide sequence, whether the transcription factor actually binds to the sequence was investigated. The binding test of the transcription factor was performed by gel mobility shift assay (EMSA).

The EMSA was carried out as follows:
Probes
EBS-1 WT (from -89 to -65): CCCCGCGCCGCCGGAAGTGT (SEQ ID NO: 6)
EBS-1 MT (from -89 to -65): CCCCGCGCCGCCGTAAGTGT (SEQ ID NO: 7)
Probes for EMSA were prepared by annealing sense chain and antisense chain of 25 nucleotide sequences at 90°C for 10 min.

Subsequently, T4 nucleatase kinase, buffer, and γ³²P were added and the mixed solution was reacted at room temperature for 30 minutes. The reaction solution was passed through a MicroSpin G-25 column (Amersham Pharmacia Biotech Corp.) and the fractions collected from the column were separated by centrifugation to obtain labeled probes. The radioactivity of the probes used was 30000 cpm or more per 1 µl.

Ten µg of protein, a reaction buffer, and probes were mixed and reacted at room temperature for 30 minutes. In the case of a super shift, the reaction was carried out at 4°C for 1 hour prior to the reaction with the probe. The reaction solution was treated in non-denatured gel by electrophoresis at 100 v, 50 mA for 3 hours. After drying the gel, the sample was analyzed by autoradiography using a Fuji BAS2000.

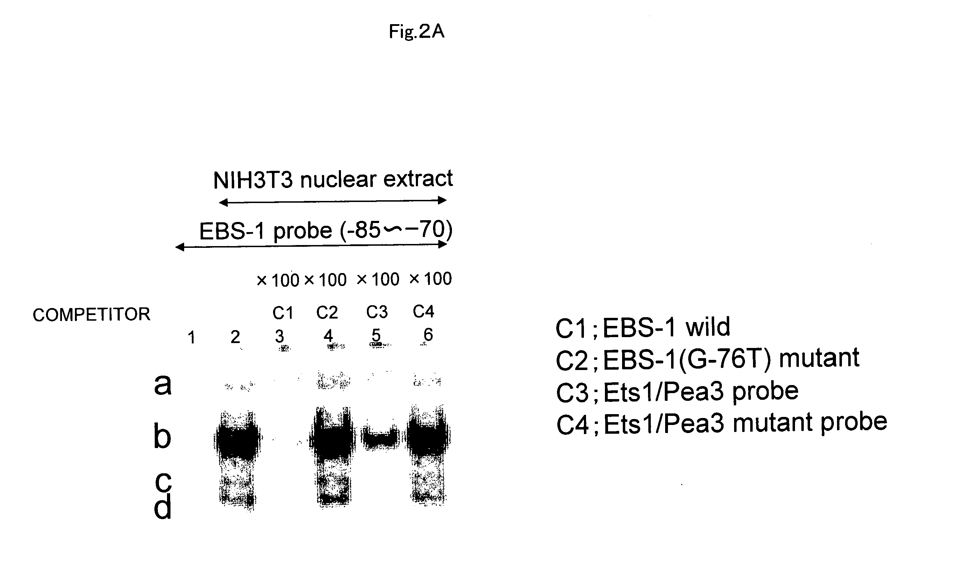

The results showed that four bands were found to be formed by gel shift using a nucleus extraction ofNIH3T3 (Bands at locations a, b, c and d in Fig. 2A). Furthermore, all these bands disappeared by cold competition (a competitive test using a non-labeled probe), but the inhibition was not found when using a probe wherein 1 nucleotide mutation has been introduced (Fig. 2A).

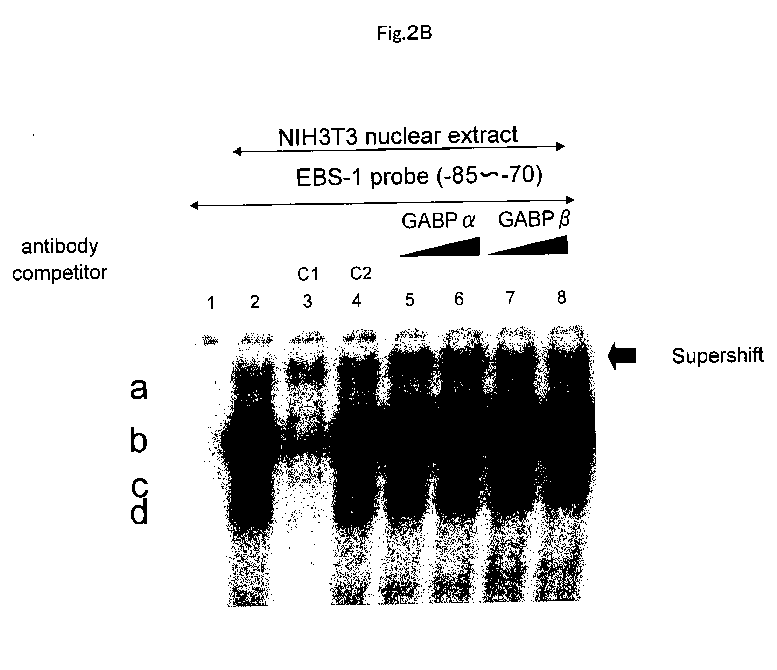

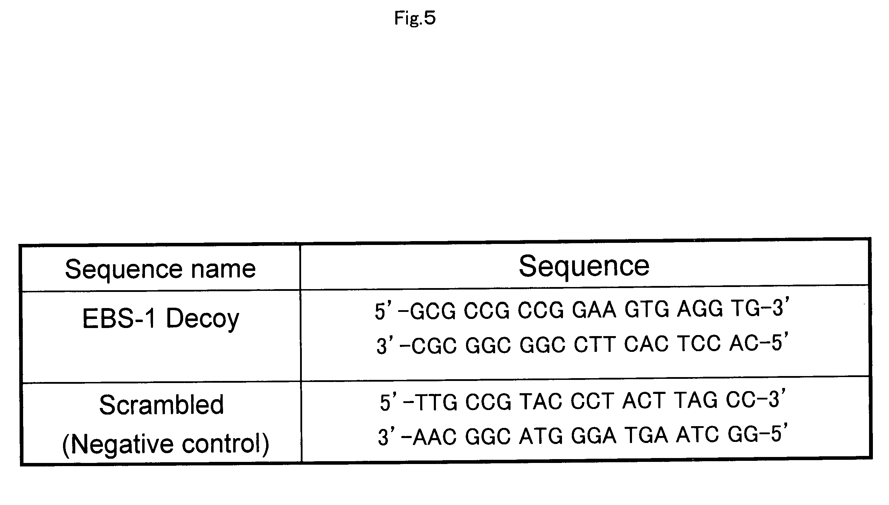

Next, in order to investigate which Ets families are bound, a cold competition of Ets 1/Pea3 and Ets probe was performed and competitive inhibition with Ets 1/Pea3 occurred such that its block disappeared by the mutation (Fig. 2B, 5^{th} and 6^{th} lanes).

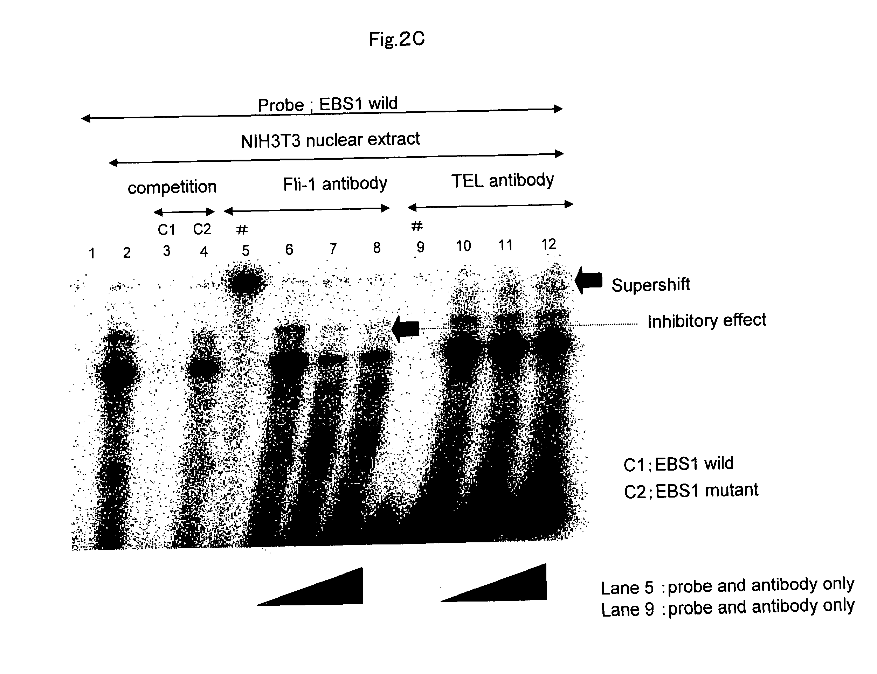

This suggested a possibility that there is transcription factors such as Ets 1, Pea 3, GABPα and the like binding to the Ets 1/Pea 3 probe (Santa Cruz Corp.). When a super shift assay was conducted using a variety of antibodies, GABPα and Tel demonstrated super shift, whereas Fli-1 indicated an inhibitory effect (Fig. 2B 5^{th} and 6^{th} lanes, Fig. 2C 6, 7, 8 lanes and 10, 11, 12 lanes). In addition, when a gel shift assay was carried out using the in vitro translation products of GABPα, Fli-1, and Ets 1, super shifts were found in all cases. This suggested that multiple Ets families are bound to this sequence.

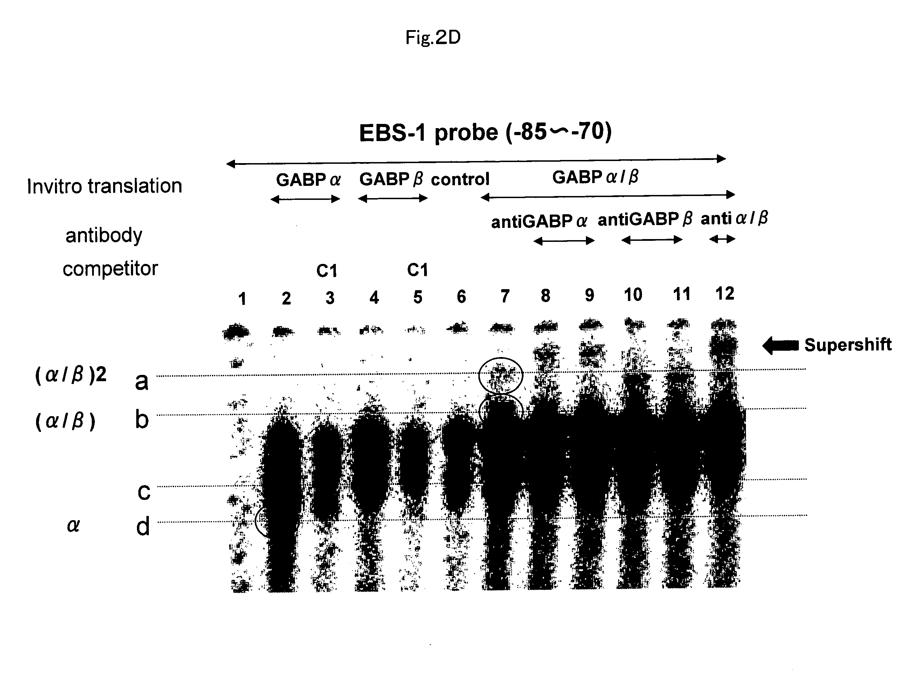

Next, complex formation of GABPβ with super shifted GABPα was investigated. Using respective in vitro translation products, a gel shift test was carried out. In the lane 7 where GABPβ protein was added to GABPα protein, formation of new bands a and b (complex) was detected (Fig. 2D). Moreover, when GABPα antibody was added, the complex a and b disappeared, resulting in super shifts (Fig. 2D, lanes 8 and 9). When GABPβ antibody was added, the formation of complex a was blocked (Fig. 2D, lanes 10 and 11).

These results suggested that GABPα/β forms a complex at EBS-1 of the synoviolin promoter (Fig. 2D).

### [Example 4]

This example confirmed the effects on the transcriptional control by Ets family in NIH3T3 and synoviolin transcription activity of GABPα and Fli-1 and Ets1.

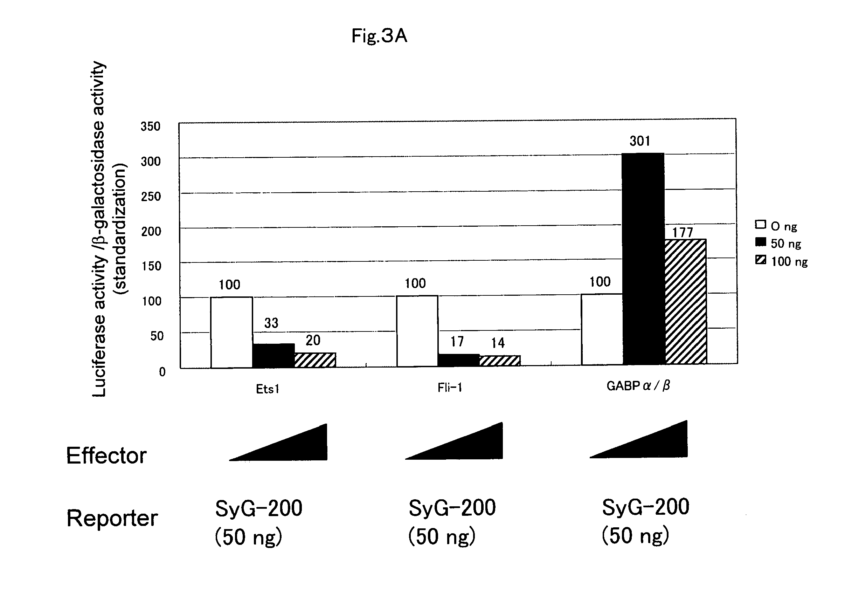

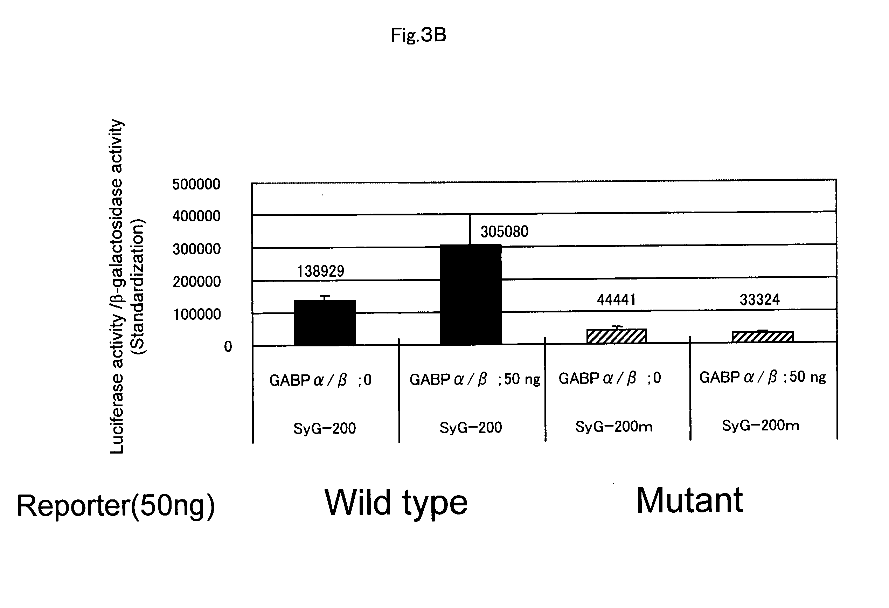

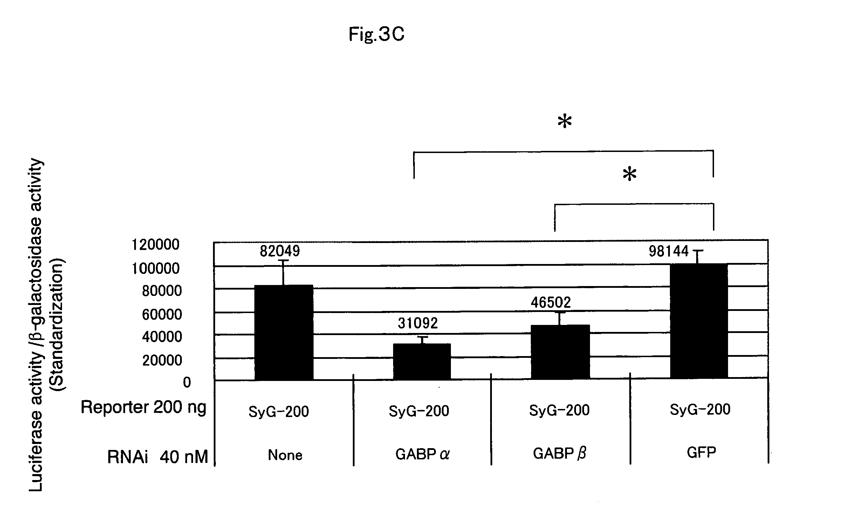

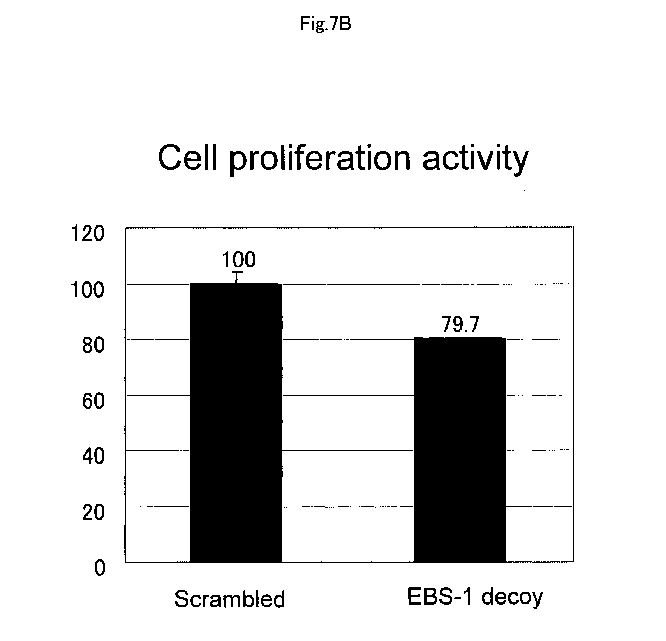

In order to evaluate the intracellular synoviolin transcription activation ability of the transcription factors binding to EBS-1, the following transcription activation assay was carried out.
(1) Oligo-preparation of ODN and preparation of cell extraction solution
   Oligodeoxyribonucleotide with a length of 20 nucleotides (ODN) was obtained by chemical synthesis. Decoy ODN was prepared by the annealing of sense and anti-sense oligonucleotides. The cells at the logarithmic proliferation stage were treated with trypsin and transferred to a 96-well plate (1 x 10³ cells/well). After 24 hours, 20 pmol of decoy ODN per well was placed in the well and transfection was performed for 3 days using LipofectAMINE (Invitrogen Corp., San Diego, CA) according to the manual attached to the kit. In order to determine the proliferation of cells having decoy ODN, a cell proliferation assay was performed using Alamar Blue (Biosource International Corp.) according to the manual attached to the kit.
(2) Oligo-preparation of RNAi and preparation of cell extraction solution
   RNA with a length of 21 nucleotides was prepared by chemical analysis. siRNA was prepared according to the protocol proposed by Elbashir et al. (Elbashir, S.M., Nature 411: 494-498, 2001). The cells at the logarithmic proliferation stage were treated with trypsin and transferred to a 96-well plate (1 x 10³ cells/well). After 24 hours, 20 pmol of siRNA per well was placed in the well and transfection was performed for 3 days using LipofectAMINE (Invitrogen Corp., San Diego, CA) according to the manual attached to the kit. In order to determine the proliferation of siRNA cells, a cell proliferation assay was performed using Alamar Blue (Biosource International Corp.) according to the manual attached to the kit (Fig. 7B).
(3) Results
   In NIH3T3, Fli-1 was found to inhibit synoviolin transcription activity, while GABPα increased its transcription activity (Fig. 3A and B). In addition, in the NIH3T3 cells knocked out with RNAi of GABPα and GABPβ, transcription of Synoviolin was found to be reduced (Fig.3C). The results suggested that in NIH3T3, the GABP α/β complex was presumed to be responsible for the expression of Synoviolin.
   In order to prove that the GABPα/β complex is responsible for the transcription activity of Synoviolin via EBS-1, a transcription activity assay was performed using promoters (-200 to +843) having an EBS-1 (G-76T) mutant and an EBS-1 wild type.
   The results revealed that the transcription activation was increased by approximately 3 times in the wild type, whereas no activation was detected with the mutant.
(4) Western blotting

Finally, NIH3T3 was treated with 200 nM decoy and the expression of Synoviolin was evaluated by Western blotting.

The Western blotting was carried out as follows. The cell culture was recovered and dissolved in a solution containing 1% NP-40, 25 mM Tris-HCl, pH 6.8, 0.25% SDS, 0.05% 2-mercaptoethanol, and 0.1% glycerol. An aliquot of transparent cell lysate was separated on a SDS-polyacrylamide gel. The protein separated was transferred to a nitrocellulose membrane and immunoblotting was carried out using anti-synoviolin monoclonal antibodies. The antibodies that were bound were detected by peroxidase bound goat anti-mouse immunoglobulin and ECL detection system (Amersham Pharmacia Biotech Corp.).

The results indicated that the expression of Synoviolin when treated with EBS-1 wild type was reduced to about one half. According to this data, it was suggested that the transcription of Synoviolin was controlled by GABPα/β via EBS-1.

### [Example 5]

This example relates to identification of the essential site of the expression of Synoviolin in mouse embryos.

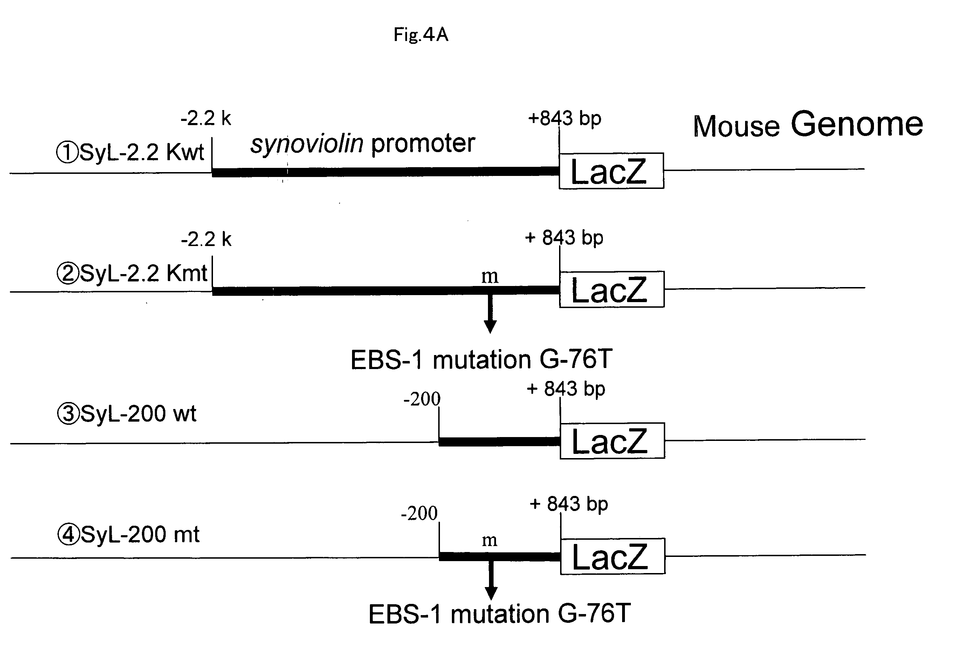

Next, in order to confirm the in vivo effects of EBS-1 in mouse embryos, a transgenic mouse was prepared by overexpressing plasmid by binding LacZ to synoviolin promoters. Four transgenic (Tg) mice were prepared by overexpressing promoters with a 2.2 k of full-length and 1 k and promoters having one-nucleotide mutation, respectively (Fig. 4A).

A construction for Tg mouse and a preparation of Tg mouse were performed as follows.

About 2.2 k fragment was cleaved with NotI and NcoI from SyG-2.2k/BV2, followed by insertion into SyTB/pBluescript to prepare SyL-2.2kwt/Bluescript. Moreover, SyL-2.2kmG-76T/pbs, SyL-200wt/pBluescript and SyL-200mG-76T/pBluescript were prepared using fragments excised from SyG. After each construction was purified using QIAGEN Plasmid Kit (QIAGEN Corp.), DNA linearized by ScaI was directly injected by microinjection into the nucleus of fertilized egg of BDF mouse (an offspring mouse after mating between C57BL/6N and DBA/2N) and then implanted to the fallopian tubes of a foster mother. Genome was extracted from each tail of the newborn mice from 8 to 9 mothers and they were confirmed to be Tg mice by Southern blotting.

In order to investigate the expression of Synoviolin from these Tg mice, X-gal staining was performed for the embryos. The expression of LacZ in the embryos from knock-out mice when LacZ was knocked in and the expressions in four Tg mice prepared in the present example were compared.

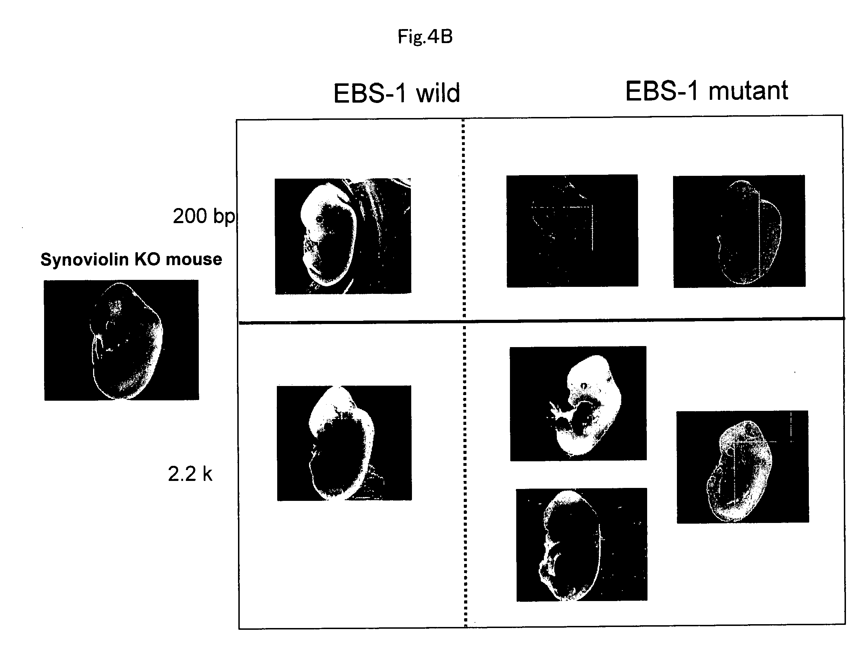

Synoviolin was stained by LacZ staining using embryos of 11.5 to 14.5 d.p.c. (days post coitus: fetal age). In the transgenic mouse where 2.2 k or 1 k promoter was introduced, staining was found at almost the same sites as those in the hetero knockout mouse. Surprisingly, it was confirmed that the expression site in the transgenic mouse where 2.2 k or 1 k promoter with 1 nucleotide mutation has been introduced was present at random (Fig. 4B) in the respective strain.

When the domain that is required for transcription activation is lost, the same results as mentioned above were reported (pax5, col11a2, etc.). From these results, it was proved that EBS-1 is an essential site for the transcription of Synoviolin.

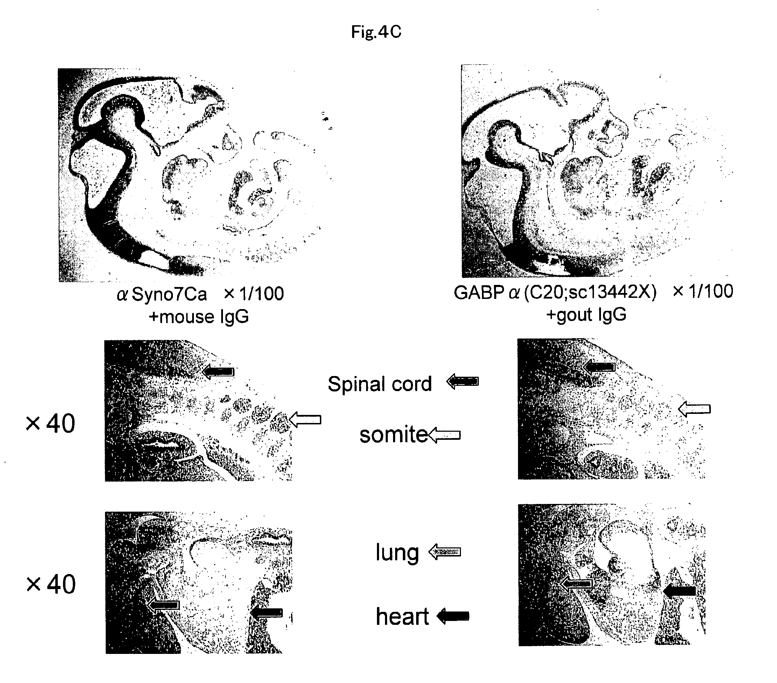

When the expression was compared to the expression of GABPα using embryos of 13.5 d.p.c., it was found to be expressed almost at the same site (Fig. 4C). According to the results, it was suggested that even during the embryonic development, as in the case of the in vitro results, GABPα controlled the expression of Synoviolin via EBS-1.

### [Example 6]

In this example, the inhibition of the expression of Synoviolin in the RA synovial cells was confirmed.

The effects of GABPα via EBS-1 in the RA synovial cells (RASC) were investigated. Even in the gel shift using the nucleus extraction solution of synovial cells, a super shift was achieved with GABPα as in the case of each extract of NIH3T3. In order to clarify the implication of EBS-1 in the RASC, namely in order to confirm that the transcriptional control of GABPα via EBS-1 leads to inhibition of the Synoviolin expression, and ultimately to inhibit hyperplasia of the synovial cells, inhibition of the expression of Synoviolin by an EBS-1 decoy was investigated.

Initially, decoy nucleic acids that bind with GABPα binding with the aforementioned EBS-1 (SEQ ID NO: 11 and 12) and decoy nucleic acids as a negative control (SEQ ID NO. 13 and 14) were designed (Fig. 5). ODNs with a length of 20 nucleotides were obtained by chemical synthesis. Decoy ODNs were prepared by annealing sense and antisense oligonucleotides.

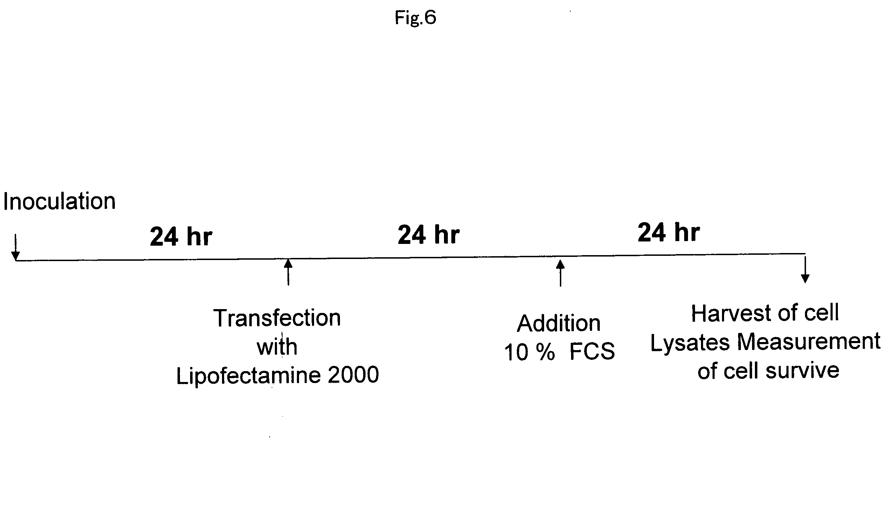

Decoy nucleic acids were transfected into cells as described below. Human RASC at the stage of logarithmic proliferation were treated with trypsin and cultured in DMEM supplemented with 10% fetal bovine serum (FCS) on a 96-well plate at 1.0 x 10³ cells/well. After 18 to 24 hours, the cells were washed with DMEM without FCS and antibiotics, and then 90 µL of DMEM without FCS and antibiotics was added. A mixture of OPTI-MEM I Reduced-Serum Medium (45 µL) and said decoy ODN (20 µM) (5µ 1) was added to a mixture of OPTI-MEM I Reduced-Serum Medium (50 µL) and Lipofectamine 2000 (1 µL) and after slightly pipetting, the mixture was left standing for 10 minutes. This was added homogeneously (10 µL) to the cells. After culturing for 24 hours, FCS (10 µL) was added and the mixture was stirred lightly and left standing to proceed with transfection for 3 days (Fig. 6). Using these cells, a cell proliferation assay was performed according to the manual attached to the kit.

For detection of horseradish peroxidase (HRP) activity, an ECL Plus kit (Amersham Corp.) was used.

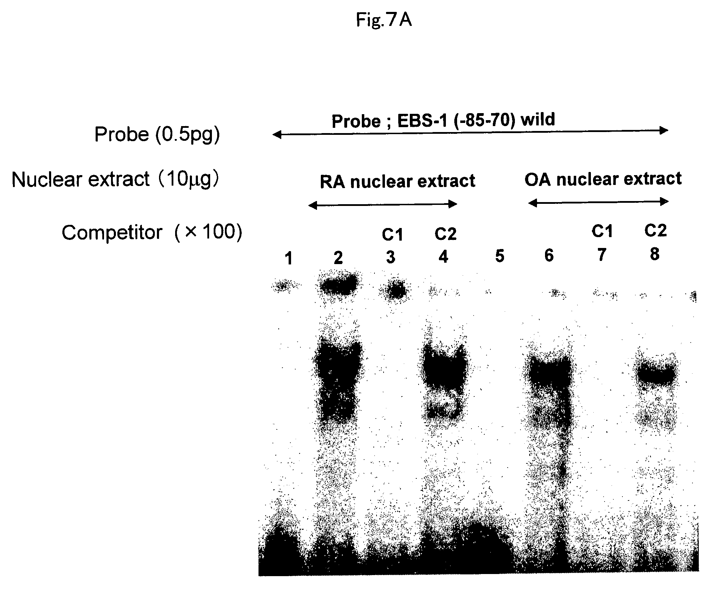

The results showed that the expression was reduced to about one half (Fig. 7A).

After culturing the aforementioned cells for 24 hours, a WST-8 assay was performed and the cells were also dissolved in the cell disrupted solution after 24 hours and Western blotting was performed. A WST-8 assay was carried out using Cell Counting Kit-8 (Dojindo Corp.). The Cell Counting Kit-8 (WST-8) was added to the cells after incubation for 24 hours and the absorbance (450 nm) was measured after 4 hours. The Western blotting was carried out as follows. After the preparation of a mixture of cell disrupted solution containing 15 mM Tris (pH 7.5), 200 mM NaCl, 0.5% NP40, 0.1 % SDS, 1 mM PMSF, 2 µg/mL leupeptin, 2 µg/mL aprotinin, and 2 µg/mL pepstatin, a protein was separated by SDS-PAGE and transcribed on a nitrocellulose (NC) membrane by an electroblotting method. This NC membrane was treated for blocking with TBS supplemented with 0.03% Tween 20 containing 5% skim milk at room temperature for 1 hour, and then anti-synoviolin antibodies or anti-β-actin antibodies were immunoreacted with TBS supplemented with 0.03% Tween 20 containing 0.5% skim milk at room temperature for 1 hour. After the reaction, the NC membrane was washed with 0.03% Tween 20/TBS and HRP labeled anti-rabbit IgG antibody was immunoreacted as a secondary antibody at room temperature for 1 hour. Subsequently, after washing with 0.03% Tween 20/TBS, HRP activity was observed to detect the target antigen.

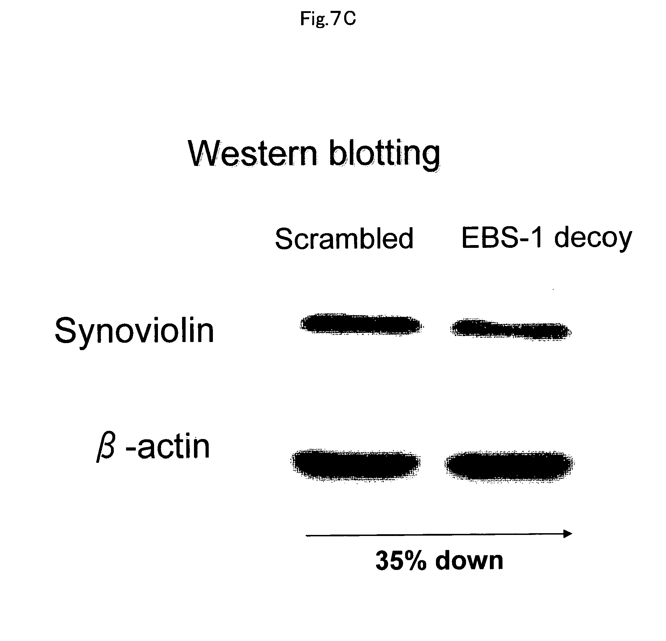

The results of the WST-8 assay are shown in Fig. 7B and the results of Western blotting are shown in Fig. 7C. As shown in Fig. 7B, when compared to the scramble decoy nucleic acid-introduced cells (a negative control), cell proliferation activity was inhibited more intensively in the EBS-1 decoy nucleic acid-introduced cells. As seen in Fig. 7C, a reduction in the amount of the production of Synoviolin protein was also confirmed by Western blotting.

As mentioned above, in the RASC, the promoter activity was found to be inhibited when said decoy nucleic acid was used.

### [Example 7]

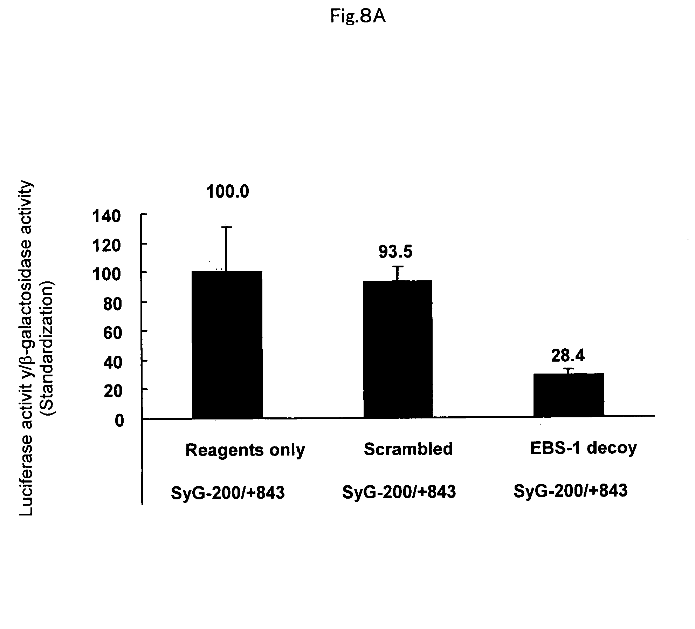

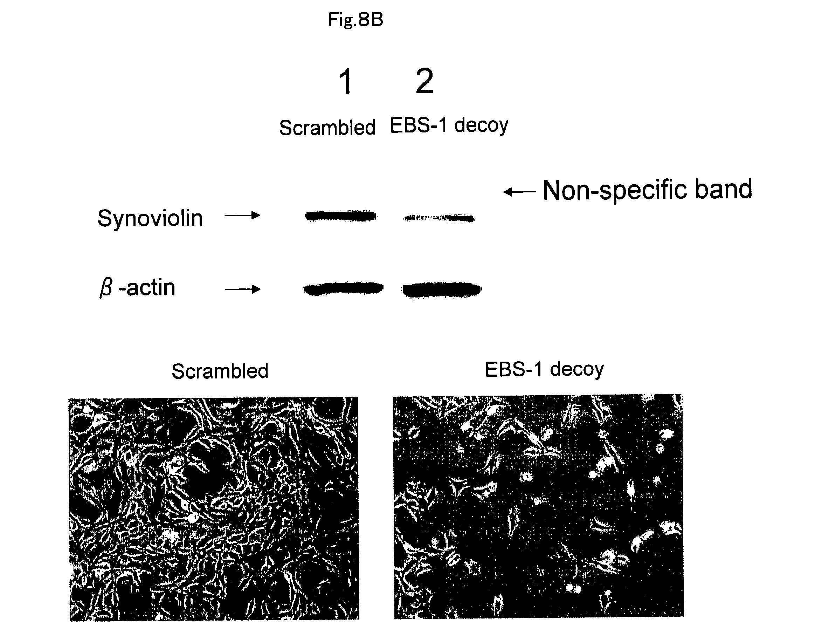

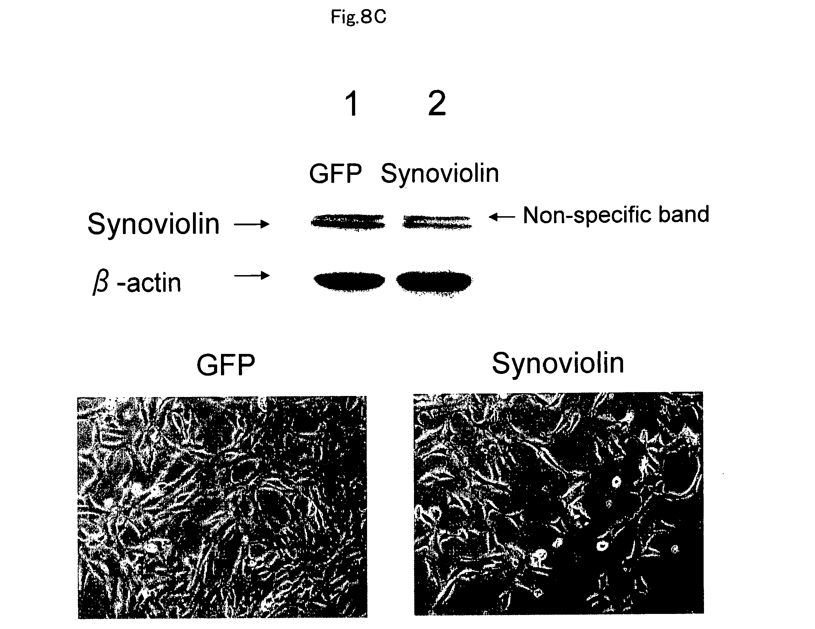

This example is to confirm the functional effects of EBS-1 in the expression of Synoviolin.
(1) Luciferase assay
   We investigated the functional effects of EBS-1 in the expression of Synoviolin. Initially, a decoy ODN targeted EBS-1 (-77/-72) (hereinafter referred to as "EBS-1 decoy") was synthesized and this EBS-1 decoy was transfected to NIH3T3 cells. The effect of EBS-1 decoy on the regulation of Synoviolin was confirmed by a Luciferase assay. Prior to the assay, it was confirmed that the effect of EBS-1 decoy transfected to the NIH3T3 cells was more than 80%, according to the immunochemical analysis using fluorescein isothiocyanate (FITC) .
   In order to perform temporary transfection to the NIH3T3 cells, a sample plasmid (100 ng) and endogenous control DNA (CMV-β-galactosidase expressed vector 50 ng) were transfected using FUGENE6™ (Roche Corp.) according to the manual (Roche Corp.) and then added to a 24-well plate. After culturing for 30 hours, the culture solution was aspirated and washed with PBS, and 100 µL of Passive Lysis Buffer™ (Promega Corp.) was added to the cells. The residue of the cell lysate was precipitated in the form of pellets and a supernatant was recovered. The Luciferase activity and the β-gal activity were measured immediately. In order to measure the Luciferase activity in the cell extract, the cell lysate (20µL) was added to an assay buffer 100 µL (0.25 mM ATP, 10 mM MgCl₂, 100 mM potassium phosphate, pH 7.8). The emission was measured with MicoLumat Plus (Perkin Elmer-Cetus Corp.). β-gal assay was carried out as follows. That is, 100 µL of an assay buffer (60 mM Na₂HPO₄, 40 mM NaH₂PO₄, 1 mM MgCl₂, 50 mM β-mercaptoethanol, 0.665 mg/ml σ-nitrophenyl-β-D galactoside) was added to the 7 µL of cell extract, and the mixture was incubated at 37° C for 30 min. 1.0 M Na₂CO₃ (160 µL) was added to stop the reaction and the absorption at 420 nm was measured. The Luciferase measurement values were all standardized (corrected) by the transfection efficiency relative to the β-galactosidase expressed by plasmid CMV-β-galactosidase and the values obtained are expressed as relative Luciferase activities. These values were further calculated relative to the SyG-200/+843 to measure mean values.
   In the Luciferase assay using the extract of the cells after introducing EBS-1 decoy, the activity was reduced to 28.4% with EBS-1 decoy when compared to the case using only the transfection reagent, indicating that transcription was inhibited (Fig. 8A).
(2) Staining by Annexin V
   The NIH3T3 cells were treated with trypsin and washed with cold PBS twice, and then suspended in 1 x Annexin binding buffer (Vybrant Apoptosis Assay Kit, Invitrogen Corp.) to be adjusted to have a concentration of 1 x 10⁶ cells/ml. 100 µl of cell suspension was used for a single test. 5 µg of FITC labeled Annexin V and 1 µl of PI calibration solution were added and the mixture was incubated at room temperature for 15 minutes. Subsequently, 400 µl of 1 x Annexin binding buffer was added and after stirring slowly, the solution was analyzed by FACSCalibur (Becton Dickison Corp.).
   Since Synoviolin exhibits resistance to apoptosis induced by ER stress, the following experiment was conducted in order to clarify the relationships between the amount of Synoviolin and apoptosis.
   The NIH3T3 cells were prepared and after 24 hours, GFP or siRNA of Synoviolin (25 nM) was transfected into the NIH3T3 cells using Lipofectamine 2000 (Invitrogen Corp.) followed by incubation for 84 hours. In addition, EBS-1 decoy ODN was prepared as described above and was transfected also into the NIH3T3 cells.
   As a result, the inhibitory effects on transcription of Synoviolin were investigated using EBS-1 decoy nucleic acid and apoptosis was found to be induced in the NIH3T3 cells (Fig. 8B). In Fig. 8B, the upper panel shows the results of Western blotting and the lower panel shows a micrograph of the NIH3T3 cells (magnification of 100 times). Similarly when the amount of the expression of Synoviolin was reduced by RNAi of Synoviolin in the NIH3T3 cells, apoptosis was found to be induced (Fig. 8C). In Fig. 8C, the upper panel shows the results of Western blotting and the lower panel shows a micrograph of the NIH3T3 cells (magnification of 100 times).
(3) FACS analysis

Moreover, the NIH3T3 cells overexpressing Synoviolin were prepared and the effect of EBS-1 decoy nucleic acid was investigated.

Stable cell lines overexpressing Synoviolin were established as follows. After 24 hours from transfection by Lipofectamine 2000 to the NIH3T3 cells, the cells were subcultured with a fresh proliferation medium by 10x dilution. Next day, a selected culture medium (supplemented with G418 0.5 µg/ml) was added and clones stably expressing HA-Synoviolin-HAHA/pc DNA3 expression vector were obtained. As a control, HA-pcDNA3 free vector was used. In order to confirm the expression from the plasmid in each cell line, Western blotting was performed using antibodies against HA tags.

The following experiment was carried out regarding apoptosis induction by EBS-1 decoy ODN.

After 84 hours of transfection of EBS-1 decoy using FUGENE6™, the cells were recovered and placed in a microtube which was labeled with Annexin V-FITC. A distribution status between live cells and cells that underwent apoptosis was measured by FACS analysis. In addition, Western blotting using Synoviolin antibodies was also carried out. As a control, β-actin antibody was used and HA-antibody was used for confirmation of stable expression.

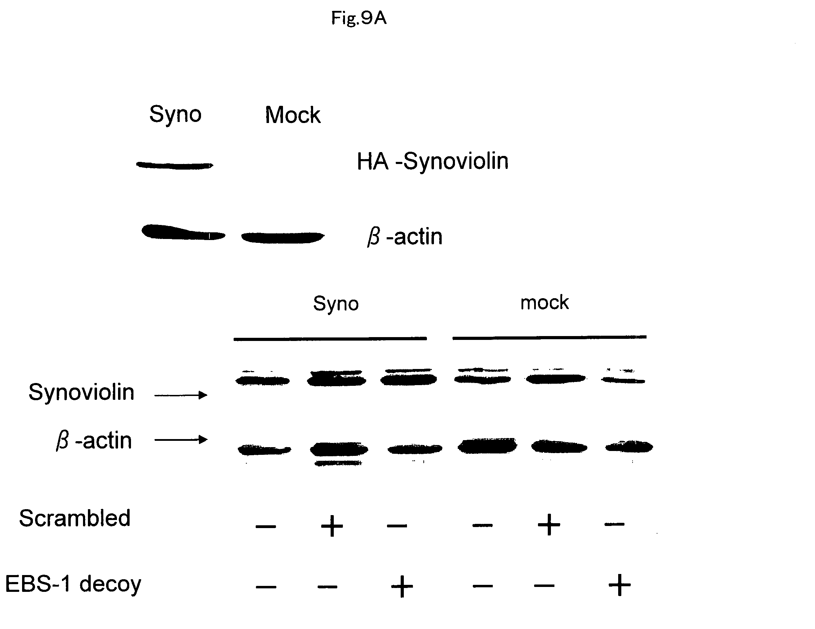

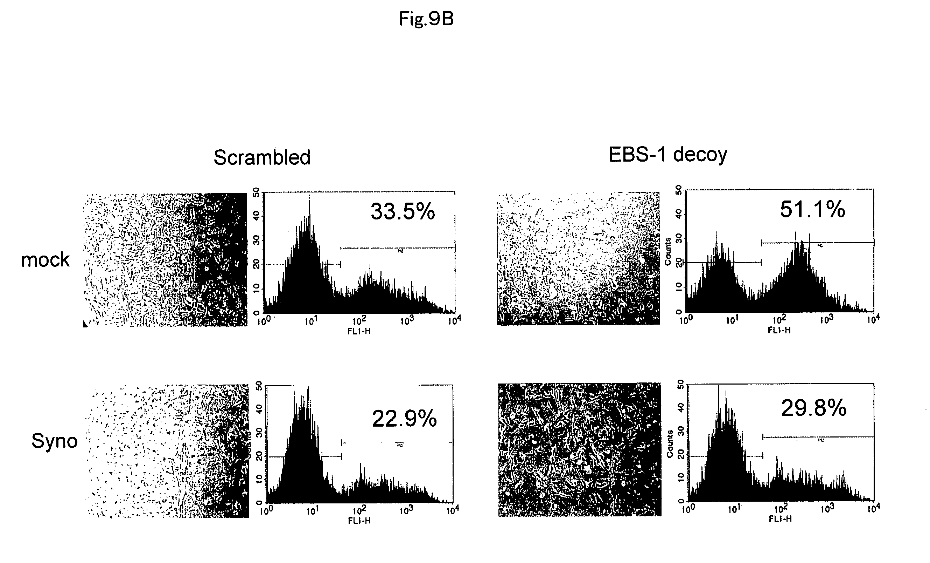

The results are shown in Fig. 9A and Fig. 9B. In the cells showing an overexpression of Synoviolin, resistance to apoptosis by EBS-1 decoy was acquired (Fig. 9A and 9B). In addition, quantification of apoptosis by FACS using Annexin V was performed. As a result, in the normal NIH3T3 cells, apoptosis was found to be 51.1%, but the ratio of apoptosis was only 29.8% in the cells showing overexpression of Synoviolin (Fig. 9B). Among the panel for the scramble and that for the EBS-1 decoy in Fig. 9B, the photograph on the left is a micrograph of the NIH3T3 cells enlarged at a magnification of 100 times.

As mentioned above, when the transcriptional regulation is controlled via EBS-1, the expression of Synoviolin is inhibited and apoptosis of the NIT3T3 cells is induced.

### [Example 8]

This example is to confirm that EBS-1 decoy inhibits the expression of synoviolin mRNA by real time PCR.

Human rheumatoid arthritis synovial cells (RASCs) at the stage of logarithmic proliferation were treated with trypsin and cultured in DMEM supplemented with 10% FCS on a 6-well plate at 1.5 x 10⁴ cells/well. After 18 to 24 hours, the cells were washed once with DMEM supplemented with FCS but without antibiotics and then 1000 µL of DMEM without FCS and antibiotics was added. A mixture consisting of OPTI-MEN I Reduced-Serum Medium (47.5 µL), said EBS-1 decoy ODN (final 100µM) (2.5 µl) or Synoviolin siRNA (final 20 µM) (2.5 µl) was combined with a mixture of OPTI-MEN I Reduced-Serum Medium (49 µL) and Lipofectamine 2000 (1 µL) and the mixture was gently pipetted and left standing for 10 minutes. The mixture was added to the cells homogeneously (100 µL each). After 24 hours, the mixture was lightly stirred and left standing, and transfection was performed by the following method.

After adding a transfection reagent for 84 hours, total RNAs were extracted from the cells by a phenol extraction method using Isogene (Nippon Gene Corp.) and RT (real time)-PCR was carried out.

The reagents used were TaqMan Universal PCR Master Mix (Roche Corp.) and the following probes purchased from Applied Biosystems Corp. were used:
Synoviolin: HS00381211_ml HRD1
hsGAPDH: Human GAPDH

That is, a 0.5 µL probe, 1 µL RNA, 5 µL TaqMan Universal PCR Master Mix and 3.5 µL purified water were placed in a PCR tube, and the mixture was blended. RT-PCR was carried out using Applied Bio RT-PCR 7500.

Cycles are scheduled as follows. One cycle of cDNA extension reaction was carried out at 50°C for 2 min and at 95°C for 10 min. Subsequently, 50 cycles of PCR amplification reaction at 95°C for 15 sec. and at 60°C for 1 min was carried out. Finally, the last extension reaction was carried out at 72°C for 5 min. and then stored at 4°C.

The expression of the amount of mRNA was analyzed using analytical software attached to the real time PCR equipment. The amount of expression of the synoviolin gene was divided by the amount of expression of hsGAPDH to correct values.

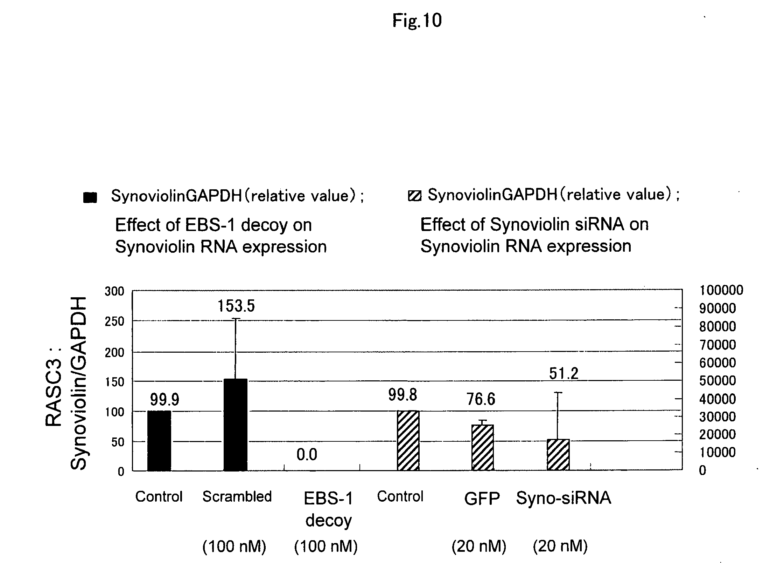

The results showed that when using EBS-1 decoy, the expression of synoviolin mRNA was found to be suppressed (Fig. 10).

### Industrial Applicability

The present invention provides decoy nucleic acids regarding promoters of the synoviolin gene. The decoy nucleic acids of the present invention can bind competitively with a transcription factor at the transcription factor binding site of Synoviolin. As a result, the activity of synoviolin promoters can be suppressed. This implies that the decoy nucleic acids of the present invention are useful for treating various diseases including RA and the like.

Sequence table free text
SEQ ID NO: 3: Synthetic DNA
SEQ ID NO: 4: Synthetic DNA
SEQ ID NO: 5: Synthetic DNA
SEQ ID NO: 6: Synthetic DNA
SEQ ID NO: 7: Synthetic DNA
SEQ ID NO: 11: Synthetic DNA
SEQ ID NO: 12: Synthetic DNA
SEQ ID NO: 13: Synthetic DNA
SEQ ID NO: 14: Synthetic DNA

## Claims

1. A decoy nucleic acid, which can inhibit promoter activity by binding to the transcription factor of the Synoviolin gene promoter.

2. A decoy nucleic acid selected from the following (a) or (b):
(a) A decoy nucleic acid consisting of a nucleic acid sequence as shown in SEQ ID NO: 11 or 12; or
(b) A decoy nucleic acid consisting of a nucleic acid sequence as shown in SEQ ID NO: 11 or 12 having deletion, substitution or addition of one or several nucleic acids, and has a function of inhibiting Synoviolin gene promoter activity.

3. A decoy nucleic acid selected from the following (a) or (b):
(a) A decoy nucleic acid consisting of a nucleic acid sequence as shown in SEQ ID NO: 11 and 12; or
(b) A decoy nucleic acid consisting of a nucleic acid sequence as shown in SEQ ID NO: 11 and 12 having deletion, substitution or addition of one or several nucleic acids, and has a function of inhibiting Synoviolin gene promoter activity.

4. The nucleic acid according to claim 2 or 3, wherein the function of inhibiting the Synoviolin gene promoter activity is a function of binding with a transcription factor of the Synoviolin gene promoter.

5. The nucleic acid according to any one of claims 1 to 4, which is designed based on a nucleotide sequence at the transcription factor binding site selected from a group consisting ofEBS, SBS and ABS.

6. The nucleic acid according to any one of claims 1 to 5, which is able to induce apoptosis in a synovial cell or a cancer cell.

7. A pharmaceutical composition containing the nucleic acid according to any one of claims 1 to 6 for treating and preventing diseases attributed to the expression of the Synoviolin gene.

8. The pharmaceutical composition according to claim 7, further containing a pharmaceutically acceptable carrier.

9. The pharmaceutical composition according to claim 7 or 8, wherein the disease is at least one selected from the group consisting of rheumatoid arthritis, fibrosis, cancers, and cerebral and neural diseases.

10. A method of inhibiting the transcription activity of the Synoviolin transcription factor using the nucleic acid according to any one of claims 1 to 6.

11. A method of inhibiting the Synoviolin promoter activity using the nucleic acid according to any one of claims 1 to 6.

12. A method of suppressing the expression of Synoviolin by inhibiting the Synoviolin promoter activity using the nucleic acid according to any one of claims 1 to 6.

13. A method of inducing apoptosis in a synovial cell or a cancer cell using the nucleic acid according to any one of claims 1 to 6.


SEQUENCE LISTING

<110> Locomogene, Inc.

<120> Decoy nucleic acid to synoviolin gene promoter

<130> G06-0055

<150> JP 2004-92570
<151> 2004-03-26

<160> 14

<170> PatentIn version 3.2

<210> 1
<211> 3046
<212> DNA
<213> Mus musculus

<400> 1
gcaagagacc ttattttgtt tttcgagaca gggtttctct gtgtagccct ggctgtccta 60

gaactcactc tgtagaccag gctggcctcg aactcagaaa tccgcctgcc tctgcctccc 120

gagtgctggg attaaaggta ggcgccacca cgcccagctt tttttttttt agataggatc 180

tcactctata gctgtacgct ggcctcagat ttatgatgct cttcctgcct cagtctccca 240

attttctggg attgtaggag tgggccacta tgctctgctc actacatgat ttcagaggtt 300

gagtagacct gaactgaaga ccagacaagg gagccctccc tcgacatctt ggggccaggg 360

aagttgaagc cataggatca gaggaaatgt ggcaagaaaa aaggccaaca tggacacaga 420

acttaaataa aaacagacag aggaagtaag acagatatat acctggggga gaggagggat 480

tgccacaaaa tgtaggagat tttcaagaat gggggaggat gagtgtgtag ggttaaaggt 540

agccagtaga agttcatagc tagccttatg gaggaaggaa aggggagcca tctcgggatg 600

ttaactgtta aagacaacag gtggtggtga agatggctga gaccaagagc acagggctga 660

ggggcagaca ggcactgaca ctgctaccct ttaatacagt tcctcctgtt gtgatcccca 720

accataatta cttcgttgct acttcataac tgtaattttg ctagttatga attgtaagta 780

aacgtctgat atgcaggata tctcatttgt gacccctgtg taacggtttg attcccaaag 840

ggcttacgac tcacaggttg agagccagcc actgccttaa agtcgtctag aatcagtttt 900

ctttcttttt tgacagacaa gatgtttaat tccgttgtac tgaaggaaag ccattttatg 960

tatttttctt aagtgctcta tcagtaatga caattctgaa agcccctgtg ttatatttta 1020

acaacacagt cacctccggt tctgtattca ctgtccgtgt tgtgactccc acagtataaa 1080

ttcctccagt tgatcttcat gaattcttat atttgatccc cccccccctt aggcctctga 1140

attccgagtg agtccgagtt aaaaatggga ggagcaccct ctagctgata aacctgggta 1200

atgaggtgtc cgctttcagt ttccattctg tacgcgacta tactgcttgt gtgagcccta 1260

acagacagaa tcagctcaga acaaagggtc tggctatctc ccagggatga acacgcacgc 1320

cgactgagct tttggggtgt tgaaaagtca acgccttcgc acagaactct ccaccccaac 1380

ctagaaataa ctggcgttct gttttatgtc agtccggaca cgcaagcact gctccttttg 1440

cgggccccgt aagcatcccc ccaggcggga tagggatccc cggcctatgg actgcgcttt 1500

ctcagctggc atccagctgc cttggcaccc agtccggggc cactctgcct acagacccta 1560

gcaaccactc acctgctttt ctttccctat aggccagaaa tttttccttt cttttctcat 1620

tggtccgcgt aactttatcg caaccaatcg gcggtacacg ggaacaaact cactcctaca 1680

caacctgcgt tggggggagg taacctggga agacctatat ctgttttctg caccgctatt 1740

tttttccgag aagcacttaa cttcttaccg tgtcgtagct atccctggaa tgaggcgctt 1800

acacatttta tttctttcat gcctgacata aagtctggcc cttgctcgct cctgcccccc 1860

gtccaaatgg ctcggcccgc ggaacgccca tcttccaggc acattgagag ccggagtctt 1920

ggagggagtt tagggtggtg attctacaac ggcgactagc aagtggcggg cttcagccct 1980

ttcccgctgc tctcctggtc gcgaccacac gtcacagctc tcgctcgttc cggttgctcg 2040

cgcagggggt ggggagtgtt gttaaccgga gcggctgccg cagtcgcggt gattgagcgt 2100

actccgccgc gccccgcgcc gccggaagtg aggtgtctta cccccgaagt tccggttcgc 2160

agggggtggg gagtgttgtt aaccggagcg gctgccgcag tcgcggtgat tgagcgtgct 2220

cgcggcgctg ggctcctggt gagtgggcct ggtcctgatt ggggttgggg ggtcggcgtc 2280

taggaccttg tcctttgggg tcactgcgat cagcccgccc cgctgcgttc ggccgccagt 2340

tttcggcctg tcagatggct ggagacctta ggcggcggcg cggccaccgt tccagaggcc 2400

gggccccgcc tgcgaggttc gcaactccta gcgttcacag gtgcgcgact gtgaggcgac 2460

ctgactggtt ctcagccccg ccgccgcacc ctggcggtcg gccgtttctc cggttctcag 2520

agtggacact gctgggggcg gggggggggg cagggttcca gactgacgta ccccgatggg 2580

cgcgcgtctg cgctgaccac cctggcacag ctgtcactgg ttgtgtcgcc ttctcaagct 2640

gtgccctctg caccttgcct cctccacccc tggcgggccc agcgaacctg cctctaaagc 2700

ctatcatccc agctccttca gagggtcagc ggtggcagcc cccctcctcc taactttgcc 2760

tcagtgactc cctagaggag gcgccttggc agacagcgtg gaagagccct agatttgaaa 2820

cgagattgat ccaagttcta ggccttgcat cagtgtgagc ctctaacccc tttgagtcct 2880

agtttctcgt ttgtgaaaca gggagtatat gctgttttga atctaatggc tgtcaaggtg 2940

aaatgagtgt ttgcccttac actctgccag ggactgtgct aggtttacat agtgtggata 3000

tcacaaatgt cattttcctt gtgcaggtct ctgggccagg gcgatg 3046

<210> 2
<211> 3092
<212> DNA
<213> Homo sapiens

<400> 2
ttggctcata acctcacttc ctttaagtct ttgctcaaat gtcaccttct caaggaagct 60

tacccgatta tcctcgctga tactgcaacc agcttcaagt accccaccac atcctgatcc 120

cctttattct gttctacttt tttcctatag cactgatcat cttccagcgt attagatttt 180

tcacttatgt ctgtggtttg ctgtcacatc tactaggata agctccacaa aggtagagat 240

ctttattttg ttcactgaca tcctaagtcc ctagaacagg agacacttga tccatatttg 300

tagactaact gaataaatga cttaattacc agtttggatg tgggggcaga tagtgagcat 360

gatgcccgtt tccggagctg gggtgcagac agtgtctagg gacactgaac tgttttaaaa 420

gcaggataga tcccggctgg agaccacaca aggaaatcat cagcacctgg gtcaggggct 480

ggactggagc agaggaaatc atgcaggaaa agtaaagaga aggacatcag gtaaagagaa 540

gaggacacat gcatagccag agagaaaaga ggagcagagg catgtggatc acagaagctt 600

agggaggaga ctttcaagaa ggggagagag gttgagtcaa gcaagggctg aaagccaacc 660

attggatgca gtcactagaa agttacagat aggcaaggtg ttgtggctca cgcctgtaat 720
cccaacacct tgtggggctg aggtgggagg atcgcttgag cccgggaggt cgaggctgca 780
atgagccctg atggcgccaa tgcactccag cctgggcgac agagcaagac cctgtcgcaa 840
aaattaataa ataaataaat aaaaagaaaa gggggaaaaa aagttatacg tggccttacg 900
gggaagccaa ctctgactgg ttataagctg aaactgtcaa gtcaacaggt ggcagggaag 960
atggctgaga ccaacagcac agagatttag aggcagacag acctggcgcc aatcctagga 1020
caggttttgg taagcctttg aatttcaatt gccccacgtt tcgggggagg gggtagcacc 1080
ccctagctca taaaccttag tgattgatga ttaaatgaga tgacggagga aaacgcaagg 1140
cacaaagtgg atgcattagc tccattttgt taatcagcag gcttagttgg ctgcgaccca 1200
gacacgaact aaaatacagt gcagcccagg accagtgggg gtcttgctta tggctcagag 1260
ctgaacaaca catgggcagc aaaatcagac actgagatgc gggcaggcct gcgacgctga 1320
agtcaattcc tttgaacaaa cagaacactt ccgtcccaag attagcagga attaatctcc 1380
cagtctcggg tacacctggt tgtccctccc tgtcctggcg cggcaaacgt tcccggaggc 1440
cagccaggga tcactcgccc aaggactgag ctttccctac tctcagccaa ctggagcggg 1500
accagggcct aggcaacgca gctgtccgcc cctaacaacc actcacctgc tttccccttt 1560
ctataggcca gcaaaggtac attctttttc ttattgggcc gcgtaactta tcgcaaccaa 1620
tcagtggcag ccacggaccc caactcactc ccacacaact tgtgggggtg atcatggaga 1680
agacaaattt ttgttttccg catccagttc tctcagagag caccgtattt gtcaaactgt 1740
tgtgactctc cctaaatgtt taagaaaaca tttcattccc ctcaggcttg tatagtctgt 1800
ccctggccta ctccccgctc caggtggtac agcccgcaag cggctcccct tcccagctgc 1860
tcgcggggcc gagtccccca gtccgaggag gccactcagc gcaggagcca taccatctgt 1920
gactaataaa taataggggg acctccgact cccccctgtt gccttattac cttccgacca 1980
cctctcggac ctcttgccca gcccttcccc gtagacatca ccccagatac ggtggtgaca 2040
ccattgctat gggcccacgt agggcgcagt gcgagccagg gcaggacgca cttggtacga 2100
cccacgccgc gccccgcgcc gccggaagtg aggtgtctga cccccgaagt tccggttcgc 2160
agggggtggg gagtgttgtt aaccggaggg gcagccgcag tcgcgcggat tgagcgggct 2220
cgcggcgctg ggttcctggt gagtggggcg aagtctggcc cgagttgtgg ttggggtcgg 2280
gacccgaacc ttccccttga ggtctccgga gtcggcacgc ccctcagccc cgccgcacgc 2340
tttcggcctg tcagctggcc ggagacctca gacgccggtg cggccgcttt gctcaagcct 2400
gggccctgcc tgcgacgccc gcaactcctg gtgctcacag gtgcgcggcc gcgagggcga 2460
cccggctcct cccgtcccgc tgctgctctc tcccgtcccg ctgtttttgt ggtgctctga 2520
gttgacacta ctccgggggt cgggggaccc caggattcca ggctgacgtt ccccgcccgc 2580
tcccgcaggg cgggcgtccg aactgcccac cctaacacag ctgtcaccgg cgctgtcgcc 2640
tgcccagcct gctatcctct gtgccttggc tgctctcagc cctggctgcg cattcccgcc 2700
cctggagcag atttctgctg ttgcctccca ccccatcttc tccaccggag ggtcagcggt 2760
gcagctcccc ctcctccaac attgcagctt ttcctcatca cctccctaga ggaggcggct 2820
tggcaggcag cgtggaaaga gccctagatt tgaagcaaga ctgacccagg ttccaggcct 2880
tgcgtcagtg tgatcactta accccttcga gtctaatttg taaaatgggg tagcgtaagc 2940

tattctttgt ctgatgattt cgagggcgaa atgtgatttc ccccccactt tctcctatga    3000

attgaggctg tgccaggcac cgggctattt tgcacagcac gagcatcaca taagttattt    3060

tcttgcccca tgcaggtctc cgggccaggg ca                                  3092

<210>  3
<211>  19
<212>  DNA
<213>  Artificial

<220>
<223>  synthetic DNA

<400>  3
gcgccgccgt aagtgaggt                                                   19

<210>  4
<211>  20
<212>  DNA
<213>  Artificial

<220>
<223>  synthetic DNA

<400>  4
aagtgagttg tcttaccccc                                                  20

<210>  5
<211>  20
<212>  DNA
<213>  Artificial

<220>
<223>  synthetic DNA

<400>  5
actccgccaa gccccgcgcc                                                  20

<210>  6
<211>  20
<212>  DNA
<213>  Artificial

<220>
<223>  synthetic DNA

<400>  6
ccccgcgccg ccggaagtgt                                                  20

<210>  7
<211>  20
<212>  DNA
<213>  Artificial

<220>
<223>  synthetic DNA

<400>  7
ccccgcgccg ccgtaagtgt                                                  20

<210>  8
<211>  11
<212>  DNA
<213>  Homo sapiens

<400>  8
gccggaagtg a                                                           11

<210> 9
<211> 6
<212> DNA
<213> Homo sapiens

<400> 9
tgaggt 6


<210> 10
<211> 10
<212> DNA
<213> Homo sapiens

<400> 10
gccgcgcccc 10


<210> 11
<211> 20
<212> DNA
<213> Artificial

<220>
<223> synthetic DNA

<400> 11
gcgccgccgg aagtgaggtg 20


<210> 12
<211> 20
<212> DNA
<213> Artificial

<220>
<223> synthetic DNA

<400> 12
cacctcactt ccggcggcgc 20


<210> 13
<211> 20
<212> DNA
<213> Artificial

<220>
<223> synthetic DNA

<400> 13
ttgccgtacc ctacttagcc 20


<210> 14
<211> 20
<212> DNA
<213> Artificial

<220>
<223> synthetic DNA

<400> 14
ggctaagtag ggtacggcaa 20

SEQUENCE LISTING

<110>  LOCOMOGENE, INC.

<120>  DECOY NUCLEIC ACID TO SYNOVIOLIN GENE PROMOTER

<130>  N.99241 GCW

<140>  EP05727504.2
<141>  2005-03-28

<150>  PCT/JP05/06527
<151>  2005-03-28

<150>  JP 2004-92570
<151>  2004-03-26

<160>  14

<170>  PatentIn version 3.2

<210>  1
<211>  3046
<212>  DNA
<213>  Mus musculus

<400>  1
gcaagagacc ttattttgtt tttcgagaca gggtttctct gtgtagccct ggctgtccta      60

gaactcactc tgtagaccag gctggcctcg aactcagaaa tccgcctgcc tctgcctccc     120

gagtgctggg attaaaggta ggcgccacca cgcccagctt tttttttttt agataggatc     180

tcactctata gctgtacgct ggcctcagat ttatgatgct cttcctgcct cagtctccca     240

attttctggg attgtaggag tgggccacta tgctctgctc actacatgat ttcagaggtt     300

gagtagacct gaactgaaga ccagacaagg gagccctccc tcgacatctt ggggccaggg     360

aagttgaagc cataggatca gaggaaatgt ggcaagaaaa aaggccaaca tggacacaga     420

acttaaataa aaacagacag aggaagtaag acagatatat acctggggga gaggagggat     480

tgccacaaaa tgtaggagat tttcaagaat gggggaggat gagtgtgtag ggttaaaggt     540

agccagtaga agttcatagc tagccttatg gaggaaggaa aggggagcca tctcgggatg     600

ttaactgtta aagacaacag gtggtggtga agatggctga gaccaagagc acagggctga     660

ggggcagaca ggcactgaca ctgctaccct ttaatacagt tcctcctgtt gtgatcccca     720

accataatta cttcgttgct acttcataac tgtaattttg ctagttatga attgtaagta     780

aacgtctgat atgcaggata tctcatttgt gacccctgtg taacggtttg attcccaaag     840

ggcttacgac tcacaggttg agagccagcc actgccttaa agtcgtctag aatcagtttt     900

ctttcttttt tgacagacaa gatgtttaat tccgttgtac tgaaggaaag ccattttatg     960

tatttttctt aagtgctcta tcagtaatga caattctgaa agcccctgtg ttatatttta    1020

acaacacagt cacctccggt tctgtattca ctgtccgtgt tgtgactccc acagtataaa 1080

ttcctccagt tgatcttcat gaattcttat atttgatccc cccccccctt aggcctctga 1140

attccgagtg agtccgagtt aaaaatggga ggagcaccct ctagctgata aacctgggta 1200

atgaggtgtc cgctttcagt ttccattctg tacgcgacta tactgcttgt gtgagcccta 1260

acagacagaa tcagctcaga acaaagggtc tggctatctc ccagggatga acacgcacgc 1320

cgactgagct tttggggtgt tgaaaagtca acgccttcgc acagaactct ccaccccaac 1380

ctagaaataa ctggcgttct gttttatgtc agtccggaca cgcaagcact gctccttttg 1440

cgggccccgt aagcatcccc ccaggcggga tagggatccc cggcctatgg actgcgcttt 1500

ctcagctggc atccagctgc cttggcaccc agtccggggc cactctgcct acagacccta 1560

gcaaccactc acctgctttt ctttccctat aggccagaaa tttttccttt cttttctcat 1620

tggtccgcgt aactttatcg caaccaatcg gcggtacacg ggaacaaact cactcctaca 1680

caacctgcgt tggggggagg taacctggga agacctatat ctgttttctg caccgctatt 1740

tttttccgag aagcacttaa cttcttaccg tgtcgtagct atccctggaa tgaggcgctt 1800

acacatttta tttctttcat gcctgacata aagtctggcc cttgctcgct cctgcccccc 1860

gtccaaatgg ctcggcccgc ggaacgccca tcttccaggc acattgagag ccggagtctt 1920

ggagggagtt tagggtggtg attctacaac ggcgactagc aagtggcggg cttcagccct 1980

ttcccgctgc tctcctggtc gcgaccacac gtcacagctc tcgctcgttc cggttgctcg 2040

cgcagggggt ggggagtgtt gttaaccgga gcggctgccg cagtcgcggt gattgagcgt 2100

actccgccgc gccccgcgcc gccggaagtg aggtgtctta cccccgaagt tccggttcgc 2160

agggggtggg gagtgttgtt aaccggagcg gctgccgcag tcgcggtgat tgagcgtgct 2220

cgcggcgctg ggctcctggt gagtgggcct ggtcctgatt ggggttgggg ggtcggcgtc 2280

taggaccttg tcctttgggg tcactgcgat cagcccgccc cgctgcgttc ggccgccagt 2340

tttcggcctg tcagatggct ggagacctta ggcggcggcg cggccaccgt tccagaggcc 2400

gggccccgcc tgcgaggttc gcaactccta gcgttcacag gtgcgcgact gtgaggcgac 2460

ctgactggtt ctcagccccg ccgccgcacc ctggcggtcg gccgtttctc cggttctcag 2520

agtggacact gctgggggcg gggggggggg cagggttcca gactgacgta ccccgatggg 2580

cgcgcgtctg cgctgaccac cctggcacag ctgtcactgg ttgtgtcgcc ttctcaagct 2640

gtgccctctg caccttgcct cctccacccc tggcgggccc agcgaacctg cctctaaagc 2700

ctatcatccc agctccttca gagggtcagc ggtggcagcc cccctcctcc taactttgcc 2760

tcagtgactc cctagaggag gcgccttggc agacagcgtg gaagagccct agatttgaaa 2820

cgagattgat ccaagttcta ggccttgcat cagtgtgagc ctctaacccc tttgagtcct 2880

agtttctcgt ttgtgaaaca gggagtatat gctgttttga atctaatggc tgtcaaggtg 2940

aaatgagtgt ttgcccttac actctgccag ggactgtgct aggtttacat agtgtggata 3000

tcacaaatgt cattttcctt gtgcaggtct ctgggccagg gcgatg 3046

<210> 2
<211> 3092
<212> DNA
<213> Homo sapiens

<400> 2
ttggctcata acctcacttc ctttaagtct ttgctcaaat gtcaccttct caaggaagct 60

tacccgatta tcctcgctga tactgcaacc agcttcaagt accccaccac atcctgatcc 120

cctttattct gttctacttt tttcctatag cactgatcat cttccagcgt attagatttt 180

tcacttatgt ctgtggtttg ctgtcacatc tactaggata agctccacaa aggtagagat 240

ctttattttg ttcactgaca tcctaagtcc ctagaacagg agacacttga tccatatttg 300

tagactaact gaataaatga cttaattacc agtttggatg tgggggcaga tagtgagcat 360

gatgcccgtt tccggagctg gggtgcagac agtgtctagg gacactgaac tgttttaaaa 420

gcaggataga tcccggctgg agaccacaca aggaaatcat cagcacctgg gtcaggggct 480

ggactggagc agaggaaatc atgcaggaaa agtaaagaga aggacatcag gtaaagagaa 540

gaggacacat gcatagccag agagaaaaga ggagcagagg catgtggatc acagaagctt 600

agggaggaga ctttcaagaa ggggagagag gttgagtcaa gcaagggctg aaagccaacc 660

attggatgca gtcactagaa agttacagat aggcaaggtg ttgtggctca cgcctgtaat 720

cccaacacct tgtggggctg aggtgggagg atcgcttgag cccgggaggt cgaggctgca 780

atgagccctg atggcgccaa tgcactccag cctgggcgac agagcaagac cctgtcgcaa 840

aaattaataa ataaataaat aaaaagaaaa gggggaaaaa aagttatacg tggccttacg 900

gggaagccaa ctctgactgg ttataagctg aaactgtcaa gtcaacaggt ggcagggaag 960

atggctgaga ccaacagcac agagatttag aggcagacag acctggcgcc aatcctagga 1020

caggttttgg taagcctttg aatttcaatt gccccacgtt tcgggggagg gggtagcacc 1080

ccctagctca taaaccttag tgattgatga ttaaatgaga tgacggagga aaacgcaagg 1140

cacaaagtgg atgcattagc tccattttgt taatcagcag gcttagttgg ctgcgaccca 1200

gacacgaact aaaatacagt gcagcccagg accagtgggg gtcttgctta tggctcagag 1260

ctgaacaaca catgggcagc aaaatcagac actgagatgc gggcaggcct gcgacgctga 1320

agtcaattcc tttgaacaaa cagaacactt ccgtcccaag attagcagga attaatctcc 1380

cagtctcggg tacacctggt tgtccctccc tgtcctggcg cggcaaacgt tcccggaggc 1440

cagccaggga tcactcgccc aaggactgag ctttccctac tctcagccaa ctggagcggg 1500

accagggcct aggcaacgca gctgtccgcc cctaacaacc actcacctgc tttccccttt 1560

ctataggcca gcaaaggtac attctttttc ttattgggcc gcgtaactta tcgcaaccaa 1620

tcagtggcag ccacgggacc caactcactc ccacacaact tgtgggggtg atcatggaga 1680

agacaaattt ttgttttccg catccagttc tctcagagag caccgtattt gtcaaactgt 1740

tgtgactctc cctaaatgtt taagaaaaca tttcattccc ctcaggcttg tatagtctgt 1800

ccctggccta ctccccgctc caggtggtac agcccgcaag cggctcccct tcccagctgc 1860

tcgcggggcc gagtccccca gtccgaggag gccactcagc gcaggagcca taccatctgt 1920

gactaataaa taataggggg acctccgact cccccctgtt gccttattac cttccgacca 1980

cctctcggac ctcttgccca gcccttcccc gtagacatca ccccagatac ggtggtgaca 2040

ccattgctat gggcccacgt agggcgcagt gcgagccagg gcaggacgca cttggtacga 2100

cccacgccgc gccccgcgcc gccggaagtg aggtgtctga cccccgaagt tccggttcgc 2160

agggggtggg gagtgttgtt aaccggaggg gcagccgcag tcgcgcggat tgagcgggct 2220

cgcggcgctg ggttcctggt gagtggggcg aagtctggcc cgagttgtgg ttggggtcgg 2280

gacccgaacc ttccccttga ggtctccgga gtcggcacgc ccctcagccc cgccgcacgc 2340

tttcggcctg tcagctggcc ggagacctca gacgccggtg cggccgcttt gctcaagcct 2400

gggccctgcc tgcgacgccc gcaactcctg gtgctcacag gtgcgcggcc gcgagggcga 2460

cccggctcct cccgtcccgc tgctgctctc tcccgtcccg ctgtttttgt ggtgctctga 2520

gttgacacta ctccgggggt cgggggaccc caggattcca ggctgacgtt ccccgcccgc 2580

tcccgcaggg cgggcgtccg aactgcccac cctaacacag ctgtcaccgg cgctgtcgcc 2640

tgcccagcct gctatcctct gtgccttggc tgctctcagc cctggctgcg cattcccgcc 2700

cctggagcag atttctgctg ttgcctccca ccccatcttc tccaccggag ggtcagcggt 2760

gcagctcccc ctcctccaac attgcagctt ttcctcatca cctccctaga ggaggcggct 2820

tggcaggcag cgtggaaaga gccctagatt tgaagcaaga ctgacccagg ttccaggcct 2880

tgcgtcagtg tgatcactta accccttcga gtctaatttg taaaatgggg tagcgtaagc 2940

tattctttgt ctgatgattt cgagggcgaa atgtgatttc ccccccactt tctcctatga 3000

attgaggctg tgccaggcac cgggctattt tgcacagcac gagcatcaca taagttattt 3060

tcttgcccca tgcaggtctc cgggccaggg ca 3092

<210> 3
<211> 19
<212> DNA

<213>  Artificial

<220>
<223>  synthetic DNA

<400>  3
gcgccgccgt aagtgaggt                                                    19


<210>  4
<211>  20
<212>  DNA
<213>  Artificial

<220>
<223>  synthetic DNA

<400>  4
aagtgagttg tcttaccccc                                                   20


<210>  5
<211>  20
<212>  DNA
<213>  Artificial

<220>
<223>  synthetic DNA

<400>  5
actccgccaa gccccgcgcc                                                   20


<210>  6
<211>  20
<212>  DNA
<213>  Artificial

<220>
<223>  synthetic DNA

<400>  6
ccccgcgccg ccggaagtgt                                                   20


<210>  7
<211>  20
<212>  DNA
<213>  Artificial

<220>
<223>  synthetic DNA

<400>  7
ccccgcgccg ccgtaagtgt                                                   20


<210>  8
<211>  11
<212>  DNA
<213>  Homo sapiens

<400>  8
gccggaagtg a                                                            11


<210>  9
<211>  6
<212>  DNA
<213>  Homo sapiens

<400>  9
tgaggt                                                                   6


<210>  10
<211>  10
<212>  DNA
<213>  Homo sapiens

<400>  10
gccgcgcccc                                                              10


<210>  11
<211>  20
<212>  DNA
<213>  Artificial

<220>
<223>  synthetic DNA

<400>  11
gcgccgccgg aagtgaggtg                                                   20


<210>  12
<211>  20
<212>  DNA
<213>  Artificial

<220>
<223>  synthetic DNA

<400>  12
cacctcactt ccggcggcgc                                                   20


<210>  13
<211>  20
<212>  DNA
<213>  Artificial

<220>
<223>  synthetic DNA

<400>  13
ttgccgtacc ctacttagcc                                                   20


<210>  14
<211>  20

<212>  DNA
<213>  Artificial

<220>
<223>  synthetic DNA

<400>  14
ggctaagtag ggtacggcaa                                                    20